Europäisches Patentamt

European Patent Office

Office européen des brevets

(19)

(11) **EP 0 487 867 B1**

(12) **EUROPÄISCHE PATENTSCHRIFT**

| | |
|---|---|
| (45) Veröffentlichungstag und Bekanntmachung des Hinweises auf die Patenterteilung:<br>**29.05.1996  Patentblatt 1996/22** | (51) Int Cl.6: **C12M 1/36**, C12N 1/00 |

(21) Anmeldenummer: **91117048.8**

(22) Anmeldetag: **07.10.1991**

(54) **Verfahren zur Erzeugung von Zellmasse und/oder Fermentierungsprodukten unter sterilen Bedingungen sowie Vorrichtung zur Durchführung des Verfahrens**

Process and apparatus for the production of a cellular mass and/or fermentation products under sterile conditions

Procédé et appareil pour la production d'une masse cellulaire et/ou des produits de fermentation sous conditions stériles

(84) Benannte Vertragsstaaten:
**AT BE CH DE DK ES FR GB GR IT LI LU NL SE**

(30) Priorität: **23.11.1990  DE 4037325**

(43) Veröffentlichungstag der Anmeldung:
**03.06.1992  Patentblatt 1992/23**

(73) Patentinhaber: **Karl Müller GmbH & Co.**
**D-70469 Stuttgart (DE)**

(72) Erfinder: **Metz, Michael**
**W-7000 Stuttgart 1 (DE)**

(74) Vertreter: **Thiel, Christian, Dr. Dipl.-Chem. et al**
**Patentanwälte**
**Herrmann-Trentepohl, Kirschner, Grosse,**
**Bockhorni,**
**Schaeferstrasse 18**
**D-44623 Herne (DE)**

(56) Entgegenhaltungen:
| | |
|---|---|
| **EP-A- 0 051 151** | **EP-A- 0 055 382** |
| **EP-A- 0 065 895** | **FR-A- 2 021 600** |
| **FR-A- 2 259 903** | **FR-A- 2 611 742** |
| **US-A- 3 591 455** | **US-A- 4 167 450** |

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft ein Verfahren zur Erzeugung von Zellmasse und/oder Fermentierungsprodukten unter sterilen Bedingungen, bei dem Fermentierungsgemisch wenigstens zeitweise im Kreislauf geführt wird und Stoffwechselprodukte der kultivierten Zellen und gegebenenfalls Zellmasse diskontinuierlich oder kontinuierlich abgetrennt werden sowie eine Vorrichtung zur Durchführung dieses Verfahrens.

Zur Zeit werden in der biotechnischen Industrie beim Einsatz von Fermentern in der Regel ansatzweise Verfahren zur Herstellung von Fermentationslösungen, Produktion von Biomasse und zur Erzeugung von Stoffwechselprodukten angewendet. Diese ansatzweise Verfahrensweise beinhaltet normalerweise das Animpfen eines Nährmediums mit der erwünschten Kultur, die Kultivierung über einen bestimmten Zeitraum unter genau definierten Bedingungen und die Ernte des Mikroorganismus und/oder Gewinnung des erwünschten Stoffwechselproduktes.

Diese ansatzweisen Verfahren besitzen jedoch eine Reihe von Nachteilen.

So findet die Mediensterilisation i.d.R. in Einsatzkonzentration bei gefülltem Fermentationstank statt. Bei Anlagen mit Behältern größer als 1.000 l Arbeitsvolumen sind bei thermischer Sterilisation der Medien hohe Heiz- und Kühlkosten anzusetzen. Bedingt durch die lange Verweilzeit des Mediums bei Temperaturen über 80°C kommt es häufig zum Qualitätsverlust des Mediums.

Der Zeitbedarf für die Sterilisation liegt im allgemeinen bei mehreren Stunden, was ein ungünstiges Verhältnis zwischen Vorbereitungszeit und Arbeitszeit ergibt. Das Verhältnis wird umso ungünstiger, je kürzer die eigentliche Fermentierungszeit ist und erreicht bei Kurzzeitfermentationen ein Verhältnis von 1 : 1.

Das Wachstum von Mikroorganismen und lebenden Zellen im allgemeinen verläuft in der ansatzweisen Fermentation unter ungünstigen Bedingungen. Zu Beginn des Wachstums benötigen die Zellen eine Zeit (lag-Phase) zur Adaption an das Medium. In der ansatzweisen Fermentation steht zu Beginn des Prozesses der niedrigsten Keimzahl (Animpfkeimzahl) die höchste Substratkonzentration gegenüber, was in vielen Fällen zur Substrathemmung führt.

In der lag-Phase ist das Verhältnis zwischen Wachstumsstoffwechsel und Erhaltungsstoffwechsel sehr ungünstig, der Organismus zehrt Substrat, wächst jedoch nicht. Dies bedeutet schlechte Substratverwertung bezogen auf Zellmassenausbeute und/oder Katabolitbildung und/oder biochemische Transformationsprodukte.

Auch im Übergang zur nachfolgenden exponentiellen Wachstumsphase ist der Anteil des Erhaltungstoffwechsels noch hoch.

Die exponentielle Wachstumsphase stellt den optimalen Bereich für das Wachstum dar, der Anteil des Erhaltungsstoffwechsel ist gegenüber dem Anteil des Wachstumsstoffwechsel gering, der Umsatz des Substrates in Zellmasse optimal. In Batch-Ansätzen wird die exponentielle Wachstumsphase jedoch durch die ansteigende Konzentration von Stoffwechselprodukten in der Regel nach kurzer Zeit beendet, da es zu einer Produkthemmung kommt, d.h. daß die Konzentration der von den Zellen ins Medium abgegebene Stoffwechselprodukte so hoch wird, daß es zuerst zu einer Verlangsamung des Wachstums und im weiteren Verlauf zum Wachstumsstillstand kommt.

Liegt die Produkthemmkonzentration bei bestimmten Zellarten sehr niedrig oder ist die Produktbildungsrate pro Zelle sehr hoch, so kommt es sehr früh im Züchtungsverlauf zu einer Wachstums- und Metabolitproduktions-Hemmung und somit zu schlechten Ausbeuten. Die Zeit, in der größere Mengen Metabolite gebildet werden, ist aufgrund der kurzen exponentiellen Wachstumsphase ebenfalls sehr kurz. Bei der ansatzweisen Produktion von Zellmasse oder Metaboliten oder Transformationsprodukten wird die Hauptmasse in sehr kurzer Zeit am Ende der exponentiellen Wachstumsphase erzeugt. Der Fermenter hat somit nur eine sehr kurze Phase hoher Produktivität (hoher Raum-Zeit-Ausbeute).

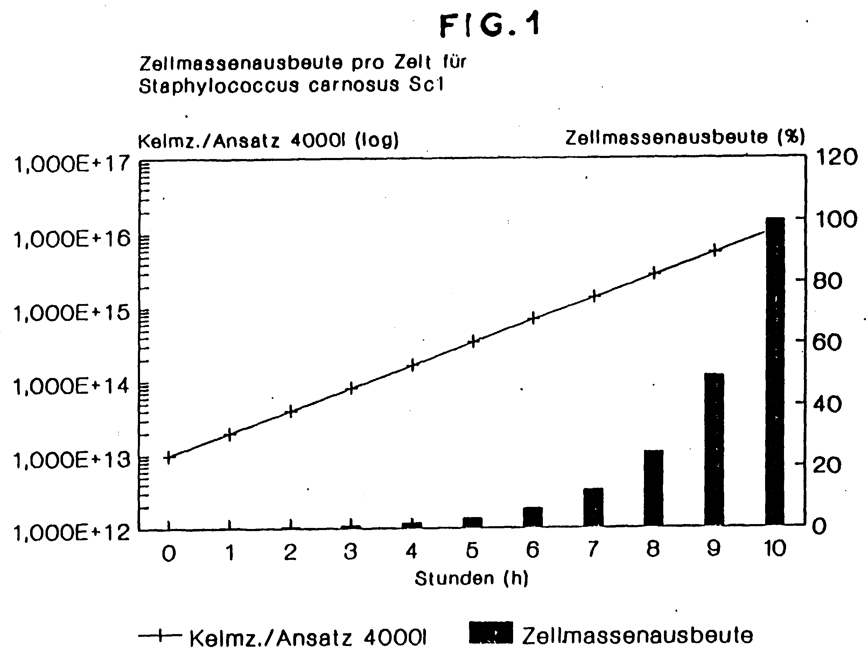

Wie aus Fig. 1 ersichtlich, wird unter den beschriebenen Verhältnissen ca. 50 % der im Prozeß gewonnenen Biomasse innerhalb einer Stunde erzeugt. Wird die gesamte Prozeßdauer, d.h. Vorbereitung - Produktion - Maschinenreinigung, mit 24 Stunden angesetzt, so wird der Fermenter nur 1/24 seiner Einsatzzeit unter optimalen Bedingungen gefahren.

Inzwischen wurden auch einige Verfahren zur kontinuierlichen Fermentation flüssiger Substrate bekannt. So beschreibt die DE-A 33 23 205 ein Verfahren und eine Einrichtung zur kontinuierlichen Fermentation eines flüssigen Substrats unter gleichzeitiger Abtrennung der im Fermentationsprozeß gebildeten Stoffwechselprodukte. Diese Arbeitsweise ist dadurch gekennzeichnet, daß das Fermentationsgemisch zu Zwecken der Fermentation im Kreislauf geführt wird, wobei innerhalb dieses Kreislaufes auch eine Membranoberfläche in dünner Strömungsschicht überströmt wird und das im Kreislauf geführte Fermentationsgemisch mit einem solche Druck beaufschlagt wird, daß während des Überströmens der Membran gleichzeitig auf dem Wege der Membranfiltration selektiv und fraktioniert die gebildeten Stoffwechselprodukte aus dem Fermentationsgemisch abgetrennt werden. Zur Aufrechterhaltung des kontinuierlichen Betriebes ist ein Substratspeicherkreis vorgeschaltet, aus dem über ein Sterilisationsmodul kontinuierlich frisches Substrat abgezogen und in den Fermentationsgemischkreislauf eingeleitet werden kann. Die Stoffwechselprodukte können kontinuierlich aus dem Prozeß abgezogen werden.

Dieses bekannte kontinuierliche Verfahren weist aber noch eine Reihe von Mängeln auf, die sowohl in konstruktiven Merkmalen als auch in Merkmalen der Verfahrensführung begründet liegen.

So arbeitet das Verfahren der DE-A 33 32 205 mit einer Membran, um die gebildeten Stoffwechselprodukte aus dem Fermentationsgemisch abzutrennen. Solche Membranen neigen aber zum Verstopfen, was die Wirksamkeit der Abtrennung herabsetzt und sehr große Membranoberflächen notwendig macht. Zudem müssen bestimmten Techniken und hohe Drücke angewandt werden, um die Membran durchlässig zu halten. Ferner erlaubt die Membran auch nicht die kontiuierliche Entschlammung der Fermentationsanlage und weiterhin auch keine kontinuierliche Abtrennung der im Fermenter gebildeten Zellmasse.

Weiterhin weist dieses bekannte Verfahren eine Reihe der Nachteile auf, die oben für das ansatzweise Verfahren geschildert werden. Diese Nachteile betreffen insbesondere die Sterilisation der Anlage und der Medien und ferner die fehlende Anpaßbarkeit der Kulturbedingungen an die jeweilige Wachstumsphase des Mikroorganismus. Dies hat eine verminderte Zellmassenausbeute und/oder Katabolitbildung bei gleichzeitig schlechter Substratverwertung zur Folge und führt zudem zu unannehmbar langen Kulturzeiten.

Aus US-A-4 167 450 ist eine Fermentierungsverfahren bekannt, bei dem die Zellmasse im Zustand des Erhaltungsstoffwechsels gehalten wird. Das Verfahren hat die Erzeugung von Stoffwechselprodukten zum Ziel, ohne daß dabei die Zellmasse erhöht wird. In dem Verfahren wird das Inokulum in einen bereits mit Substrat vollständig gefüllten Fermenter eingegeben. Um das Wachstum der Kultur im Reaktor zu begrenzen, wird die Substratkonzentration im Laufe des Verfahrens gesenkt.

EP-A-0 065 895 beschreibt eine Verfahren zur Erzeugung von Mircoorganismen, bei dem der Mikroorganismus in einer ersten Phase in einem hochkonzentrierten Nährmedium gehalten wird, dessen Konzentration zunächst konstant gehalten wird. In einer zweiten Phase wird erschöpftes Nährmedium ausgetauscht und die Nährmediumkonzentration gesenkt, um die Bildung von wachstumshemmenden Metaboliten zu unterdrücken.

FR-A-2 259 903 und FR-A-2 021 600 beschreiben kontinuierliche Fermentierungsverfahren. Bei ersterem ist die Fermentierungseinheit mit einem Kreislauf ausgerüstet, wobei ein Teil des Mediums entnommen und durch frisches Medium ersetzt wird. Bei letzterem wird der Sauerstoffpartialdruck in dem aus dem Reaktor stammenden Gas zur automatischen Steuerung der Nährstoffzuführung verwandt. Die Substratkonzentration wird hier konstant gehalten.

FR-A-2 611 742 beschreibt ein Verfahren zur Erzeugung von Microorganismen und/oder Metaboliten in einem Nährmedium, in dem die Substratkonzentration über die gesamte Verfahrensdauer konstant gehalten wird. Der Reaktor ermöglicht den Austausch von verbrauchtem Nährmedium und die ansatzweise kontinuierliche Verfahrensführung.

EP-A-0 051 151 betrifft ein Fermentierungsverfahren und dazu geeigneten Reaktor, bei denen Schaum mit Hilfe einer im Reaktor selbst angeordneten Zentrifugeneinrichtung in eine Gasphase, eine an Zellen abgereicherte flüssige Phase und eine zellreiche flüssige Phase aufgetrennt wird.

Der Erfindung liegt die Aufgabe zugrunde, ein Verfahren der eingangs geschilderten Art bereitzustellen, bei dem sowohl im kontinuierlichen als auch im semikontinuierlichen und ansatzweisen Betrieb eine optimale Anpassung der Kulturbedingungen an die Wachstumsphase eines kultivierten Mikroorganismus möglich ist, eine optimale Einstellung auf Zellmassenproduktion und/oder Katabolitbildung möglich ist und das Nährmedium für den jeweiligen Zweck optimal verwertet wird.

Diese Aufgabe wird mit dem Verfahren der eingangs geschilderten Art gelöst, welches die folgenden Schritte aufweist:

- Beschicken der Fermentationsanlage mit einer zum Starten der gewünschten Zellkultur ausreichenden Menge an Nährmedium;
- Sterilisieren der Anlage sowie Einstellen der gewünschten Nährmediumkonzentration- die niedrige ist, als die spezifische Nährmediumkonzentration der Zellkultur;
- Beimpfen des Nährmediums mit der Animpfkultur und ungestörten Wachsenlassen der Kultur über einen definierten Zeitraum in ansatzweiser Verfahrensweise und unter Teilfüllung der Fermentationsanlage;
- Steigerung der Konzentration des Nährmediums auf die spezifische Nährmediumkonzentration der Zellkultur bei gleichzeitiger Steigerung des Nährmediumvolumens auf das Arbeitsvolumen der Anlage sowie der Zellkonzentration;
- Übergang zu kontinuierlicher Verfahrensweise unter Austausch des Nährmediums und Abtrennung der Stoffwechselprodukte sowie vollständiger oder teilweiser Zellrückführung;
- Abruch der kontinuierlichen Verfahrensweise zu einem gewünschten Zeitpunkt und Ernte der Zellmasse unter sterilen Bedingungen; und gegebenenfalls
- Wiederholung eines Teils oder aller vorgenannter Schritte in der genannten Reihenfolge.

Bevorzugte Verfahrensweisen sind Gegenstand der Unteransprüche.

Das erfindungsgemäße Verfahren ist auf jeden in üblichen Fermentern kultivierbaren Mikroorganismus anwendbar. Es werden übliche Kultivierungsbedingungen und Nährmedien eingesetzt; der Vorteil des erfindungsgemäßen Verfahrens liegt in der besonderen Art der Verfahrensführung und nicht in der Anwendung besonderer Bedingungen oder Medien. Sofern erfindungsgemäß mit hohen Frequenzen gerührt oder mit einer Zentrifuge abgetrennt wird, sind nicht scherkraftempfindliche Organismen bevorzugt.

Das erfindungsgemäße Verfahren kann zur Kultivierung von Bakterien und Pilzen verschiedenster Art eingesetzt werden. Besonders geeignet ist es zur Kultivierung von Bakterien, aerober wie anaerober, gram-positiver wie gram-negativer. Besonders zu nennen sind die verschiedenen Coccen, insbesondere Mikrococcen, Planococcen, Deinococcen, Staphylococcen, Stomatococcen, Streptococcen, Leuconostoc, Pediococcen, Aerococcen, Gemella, Peptococcen, Peptostreptococcen, Ruminococcen, Cuprococcen, sowie die Gattung Sarcina. Weiterhin Bakterien der Gattungen Bacillus, Sporolactobacillus, Clostridium, Desulfotomaculum, Sporosarcina, Planococcus, Lactobacillus und Korthia. Geeignet sind ferner Bifidobakterien, Brevibakterien, Bakterien der Gattungen Zymomonas, Acetobacter, Gluconobacter, Pseudomonas, Vibrio und Aeromonas. Zu nennen sind ferner gram-negative anaerobe Bakterien der Gattungen Escherichia, Shigella, Edwardsiella, Citrobacter, Salmonella, Klebsiella, Enterobacter, Hafnia, Serratia, Proteus, Providencia, Morganella, Yersinia, Erwinia, Obesumbacterium, Kluyvera, Cedecea, Tatumella, Xenorhabdus, und Rahnella. Gram-negative aerobe Stäbchen und Kokken, die für das erfindungsgemäße Verfahren geeignet sind, sind solche der Familien Pseudomonadaceae, Azotobacteraceae, Rhizobiaceae, Methylococcaceae, Halobacteriaceae, Acetobacteraceae, Legionellaceae und Neisseriaceae.

Das erfindungsgemäße Verfahren eignet sich sowohl zur Vermehrung und Gewinnung der darin kultivierten Mikroorganismen, die aus dem Verfahren abgetrennt und anschließend anderweitig verwertet werden können, oder aber zur Gewinnung der von den Mikroorganismen erzeugten Stoffwechselprodukte. Es ermöglicht dabei in einfacher Weise, die Ausbeute an Mikroorganismus oder aber an Stoffwechselprodukt zu optimieren, d.h. einen Arbeitspunkt einzustellen, an dem die maximale Vermehrungsrate des Mikroorganismus genutzt wird, oder aber die optimale Substratumwandlung. Das erfindungsgemäße Verfahren ermöglicht also die Einstellung von Arbeitspunkten mit einem geringen Anteil des Erhaltungsstoffwechsels an der Substratnutzung, was bei der Erzeugung von Zellmasse wichtig ist, oder aber mit einem hohen Anteil des Erhaltungsstoffwechsels und geringer Zellproduktion, was bei der Gewinnung von Stoffwechselprodukten von Bedeutung ist. Des weiteren ermöglicht das Verfahren eine weitestgehende Nutzung des jeweiligen Nährmediums und zugleich eine kontinuierliche Abführung der Stoffwechselprodukte, wodurch eine Substrathemmung vermieden wird.

Die Erfindung wird durch die beigefügten Abbildungen näher erläutert, von denen

Fig.1     die Zellmassenausbeute pro Zeiteinheit für Staphylococcus carnosus Sc1;

Fig.2     einen Vergleich des Zeit- und Energiebedarfs für die Sterilisation eines 5000 l Fermenters mit unterschiedlichen Sterilisationsvolumina;

Fig.3     die Abhängigkeit der mittleren Wachstumsrate μ von der Ausgangsglucosekonzentration im Medium für Lactobacillus curvatus S3;

Fig.4     den Verlauf der spezifischen Wachstumsrate μ bei linearem und exporentiellem Zellwachstum;

Fig.5     einen erfindungsgemäßen Fermenter mit Zellrückführung über Separator;

Fig.6     die Steuerung des erfindungsgemäßen Fermenters mit Zellrückführung über einen Separator;

Fig.7     bei der Kultivierung von Pediococcus pentosaceus ermittelte Verfahrensparameter in Abhängigkeit von der Zeit;

Fig. 8     ausgewählte Parameter der bei der Kultivierung von Pediococcus pentosacirus erhaltenen Verfahrensparameter zeigt;

Fig. 9     entspricht Fig.7 für Lactobacillus curvatus Stamm 2 und

Fig. 10    entspricht Fig.8.

Das erfindungsgemäße Verfahren läßt sich in fünf Schritte gliedern, die nach dem Start der Anlage zyklisch ablaufen und in die folgenden verfahrenstechnischen Aufgaben unterteilt werden können.

1. Vorbereitung und Sterilisation der Fermentationsanlage und der Nährmediumkomponenten;

2. erster Arbeitszyklus der Anlage in ansatzweiser Verfahrensweise mit niederer Medienkonzentration und Teilfüllung;

3. nach Erreichen einer spezifischen Medienkonzentration Übergang auf geregelte Mediumdosierung bis zum Erreichen des Fermentationsendvolumens;

4. Übergang auf kontinuierliche Verfahrensweise mit Nährmediumtausch und vollständiger Zellrückführung oder mit teilweiser Zellabtrennung;

5. Abbruch des kontinuierlichen Fermentationsabschnittes und Ernte der gesamten Biomasse unter sterilen Bedingungen;

6. gegebenenfalls Neuansatz steriler Nährmedien in die sterile Anlage und Wiederholung des Prozesses gemäß der Schritte 1 - 5 unter Einsparung der gesamten Sterilisationszeit und Energie.

Die Sterilisation der gesamten Anlage in Schritt 1 erfolgt so, daß im Produktionskessel ein Mediumkonzentrat hitzesterilisiert wird. Sobald das Konzentrat nach der Sterilisation unter 100°C abgekühlt ist, wird es durch direktes Einspritzen von sterilfiltriertem kaltem Verdünnungswasser und anschließend sterilfiltrierbaren Medien innerhalb kurzer Zeit auf Fermentationstemperatur abgekühlt. Eingespart wird dadurch die thermische Energie für die Hitzesterilisation des gesamten Mediumvolumens; die Kühlenergie für die Kühlung des gesamten Fermentationsvolumens von Temperaturen von nahezu 100°C auf die Fermentationstemperatur, sofern die Mischungsverhältnisse der kalten und der heißen Phase aufeinander abgestimmt sind; und Zeit, die zur Kühlung des gesamten Fermentationsvolumens im gerührten Kessel über den Doppelmantel notwendig ist, da die für die Konzentratkühlung über Direkteinspritzung von sterilem Wasser bis zum Erreichen des Fermentationsvolumen benötigte Zeit vergleichsweise gering ist.

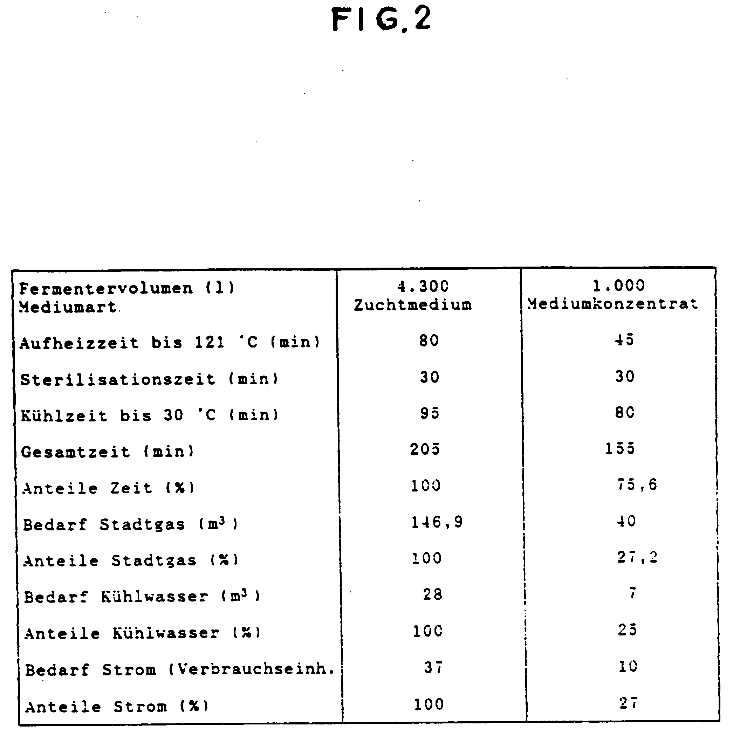

Fig.2 gibt einen Vergleich des Zeit- und Energiebedarfs für die Sterilisation eines 5000 l Fermenters mit unterschiedlichen Sterilisationsvolumina. Es werden ca. 25 % Zeit und ca. 75 % der Gas-, Wasser- und Strommenge eingespart.

Das Anfahren der Anlage in ansatzweiser Verfahrensweise mit niederer Medienkonzentration gemäß Schritt 2 ermöglicht eine optimale Anpassung der Zellen an ihr Substrat und ergibt hohe Zellwachstumsraten, da bei entsprechend niederen Medienkonzentrationen weder eine Substrat- noch eine Produkthemmung auftritt. Demgegenüber wird bei der üblichen ansatzweisen Verfahrensweise die Mediumkonzentration zur Erzielung hoher Ausbeuten hoch gewählt, was zu Beginn des Prozesses zu einer Substrathemmung führt und das Anwachsverhalten und die Zellmassen Katabolit- und Transformationsproduktausbeute verschlechtert.

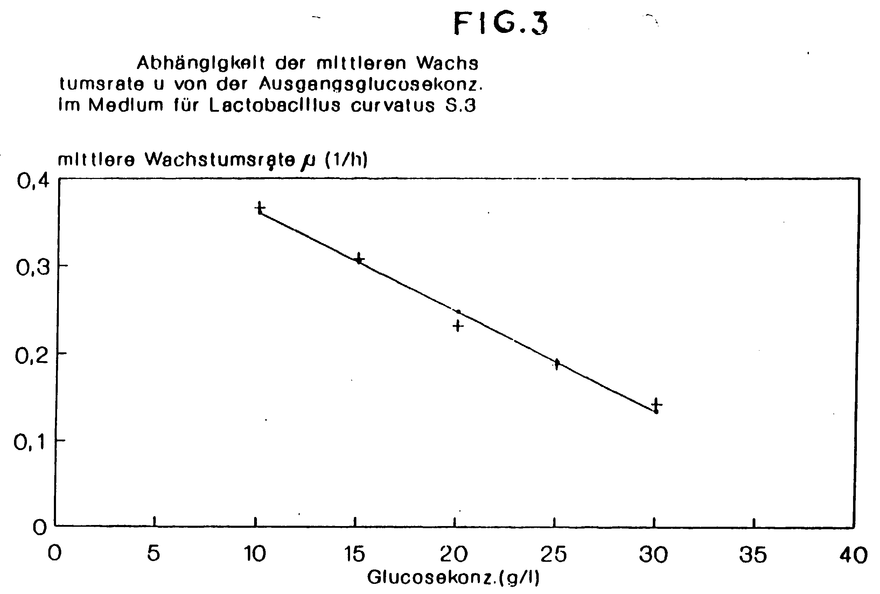

Fig.3 zeigt die Abhängigkeit zwischen der Glucosekonzentration im Zuchtmedium und der mittleren Wachstumsrate μ des Stammes Lactobacillus curvatus stellvertretend für die anderen Mikroorganismen. Je höher die Glucosekonzentration im Medium liegt, desto niedriger ist die mittlere Wachstumsrate, d.h. bei niederer Glucosekonzentration erhöht sich die mittlere Wachstumsrate.

In Schritt 3 wird in Abhängigkeit vom Verbrauch eines Leitsubstrates oder über eine vorgegebene Dosagefunktion Medium einer definierten Konzentration in den Fermenter dosiert, was zu einer Volumenzunahme führt. Die ansteigende Zellkonzentration wird durch die Mediumdosage ständig verdünnt, so daß keine Produkthemmung auftritt. Die Konzentration des zudosierten Mediums ist so eingestellt, daß bei Erreichen des Fermentationsendvolumens eine Gesamtmediumkonzentration vorliegt, bei der die Wachstumsrate noch so hoch ist, daß der %-Anteil des Wachstumsstoffwechsels gegenüber dem %-Anteil des Erhaltungsstoffwechsels eine für die jeweilige Zelle gültigen Wert nicht überschreitet.

Schritt 3 weist gegenüber der ansatzweisen Verfahrensweise den Vorteil auf, daß die Mediumsdosagemenge im Lauf des Zellwachstums ansteigt und die Stoffkonzentration im Medium im wesentlichen konstant gehalten wird. Dies bewirkt ein exponentielles Zellwachstum über den gesamten Schritt. In der herkömmlichen ansatzweisen Verfahrensweise nimmt die Substratmenge über das Zellwachstum ab, die Stoffkonzentrationen sind veränderlich. Dies wirkt dem idealen Zellwachstum entgegen. Ein weiterer Vorteil von Schritt 3 ist die sehr gute Substratnutzung in diesem Verfahrensabschnitt, bedingt durch die bedarfsorientierte Mediumdosage.

Im erfindungsgemäßen Verfahren wird in Schritt 4 bei Erreichen des Fermentationsendvolumens ein Medientausch eingeleitet. Die Abtrennung des Mediums erfolgt mittels eines sterilen Trennsystems, vorzugsweise mittels einer steril laufenden Zentrifuge. Die Zellen werden gemäß einer ersten Variante 4a vollständig in den Prozeß zurückgeführt. Das aus dem System abgetrennte, verbrauchte Mediumvolumen wird dem System in Form von frischem Medium zugeführt. Die Konzentration des zudosierten Mediums ist so gewählt, daß sie unterhalb der Konzentration liegt, die zu einer Produkthemmung führt, so daß die Zellmasse substratlimitiert wächst.

Der Übergang von der ansatzweisen zur kontinuierlichen Verfahrensweise erfolgt auf die nachfolgend beschriebene Weise, um die maximale Substratnutzung zu gewährleisten. Zu Beginn des kontinuierlichen Schrittes wird die Mediumdosagemenge aus dem spezifischen Verbrauch des Leitsubstrates oder über die Wachstumskurve errechnet, während das Mediumtauschvolumen von Beginn des kontinuierlichen Schrittes an auf der maximal möglichen Abtrennrate gehalten wird. Dies führt zu Beginn des kontinuierlichen Schrittes zu einer Verdünnung des Mediums und zu einer erhöhten Auswaschung von Stoffwechselprodukten, was das weitere Wachstum der Zellen fördert.

Im weiteren Verlauf der Züchtung steigt der Medienbedarf mit steigender Zellmasse an bis zum Erreichen der Mediendosagemenge, bei der die dosierte Medienkonzentration den optimalen kontinuierlichen Arbeitsbereich erreicht. Von diesem Zeitpunkt an wird die Mediendosagemenge konstant gehalten, das System befindet sich nun im stabilen substratlimitierten Zustand.

Die spezifische Wachstumsrate μ der Zellen, die bis zum Erreichen des optimalen kontinuierlichen Arbeitsbereichs nahezu den gleichen Wert beibehält, sinkt im Betrieb am optimalen kontinuierlichen Arbeitsbereich exponentiell ab, während die Keimzahl linear zunimmt.

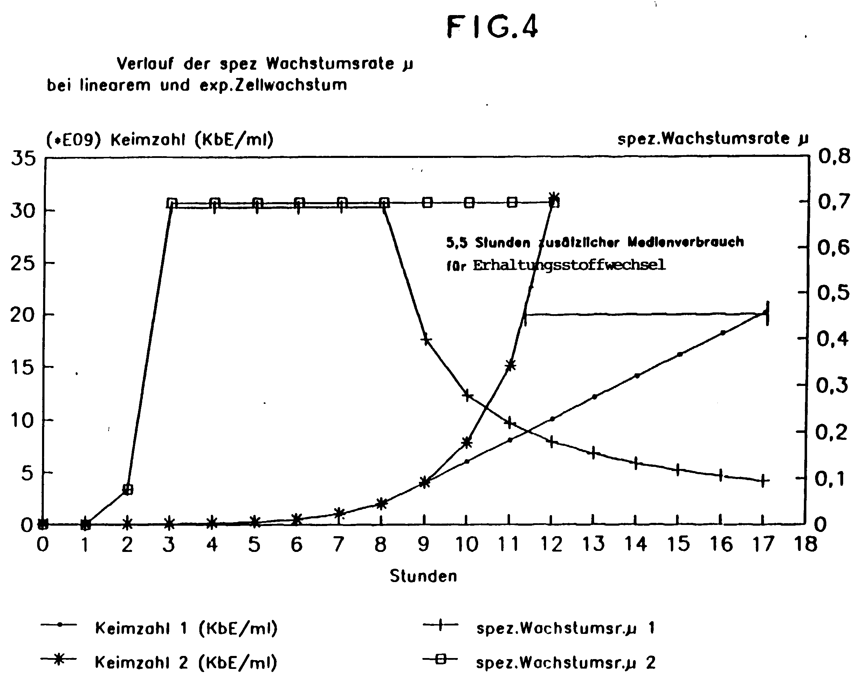

Fig. 4 zeigt den Verlauf der spezifischen Wachstumsrate μ bei linearem und exponentiellem Zellwachstum. Vergleicht man die Zeit, die ein exponentiell wachsender Organismus benötigt, um eine bestimmte Keimzahl zu erreichen mit der Zeit, die derselbe Organismus benötigt, wenn er linear wächst, so ergibt sich hierbei eine erhebliche Zeitdifferenz. Lineares Wachstum in einer Kultur findet dann statt, wenn die Nährmediumszufuhr bzw. die Verfügbarkeit des Nährmediums nicht exponentiell, sondern linear ist. Bei linearem Wachstum nimmt mit steigender Zellzahl die Wachs-

tumsrate μ exponentiell ab. Dadurch wird der Anteil des Erhaltungsstoffwechsels am ganzen Stoffwechsel sehr hoch. Experimentell wird ermittelt, bei welchem μ mit der Teilabtrennung begonnen wird.

Gemäß einer zweiten Variante 4b wird das erfindungsgemäße Verfahren in Schritt 4 unter Teilabtrennung der Mikroorganismen gefahren, etwa bei biochemischen Transformationen. Hier wird in Schritt 4 eine Zellmassenkonzentration angefahren, bei der die Wachstumsrate μ am Punkt der maximalen Transformationsrate liegt. Ist dieser Punkt erreicht, wird mit einer Zellmassenentnahme aus dem System begonnen. Die Zellkonzentration im System wird dadurch konstant gehalten. Dadurch hält man das System an einem definierten Arbeitspunkt mit maximaler Transformationsrate.

Schritt 5 mit Abbruch des kontinuierlichen Fermentationsschrittes wird in der Variante 4a dann eingeleitet, wenn die Wachstumsrate μ einen unteren Grenzwert erreicht hat. Der Grenzwert ergibt sich in der Regel aus der Stoffwechselphysiologie der Zellen, die je nach späterem Einsatz bestimmte Merkmale aufweisen müssen.

Bei der Ernte einer Züchtung gemäß 4a wird die gesamte Biomasse abgetrennt. Dieser Schritt entspricht im Prinzip einer ansatzweisen Verfahrensweise, unterscheidet sich hiervon jedoch stark in bezug auf die Zellmassenausbeute. Die Ausbeute liegt beim erfindungsgemäßen Verfahren deutlich höher als beim üblichen ansatzweisen Verfahren.

Die Ernte gemäß einer ansatzweisen Verfahrensweise (Schritt 4a) ist bei solchen Prozessen wichtig, bei denen das Produkt aus rechtlichen oder verarbeitungstechnischen Gesichtspunkten als Charge definiert werden muß, so. z. B. in der pharmazeutischen oder Lebensmittelindustrie. Das erfindungsgemäße Verfahren weist sowohl die Vorteile eines kontinuierlichen Verfahrens mit Zellrückführung im Hinblick auf Zellertrag als auch die einer eindeutigen Chargendefinition auf. Die Ernte erfolgt jeweils unter sterilen Bedingungen.

In Schritt 4b ist dagegen ein voll kontinuierliches Fermentationsverfahren beschrieben, dessen Abbruch in Schritt 5 nicht durch die Wachstumskinetik der Zellmasse, sondern durch äußere Einflüsse wie z.B. Infektion, defekte Anlagenkomponenten oder bewußten Abbruch erfolgt.

Nach der Ernte in Schritt 5 wird der produktberührte Teil der Anlage mit sterilfiltriertem Wasser gespült und mit kurz zuvor sterilisiertem Medium aus den Dosage-Tanks befüllt und ist dann sofort wieder für den nächsten Ansatz bereit. Dieser Ansatz wird wieder mit einer in der exponentiellen Wachstumsphase befindlichen Vorkultur angeimpft und der Prozeß wiederholt.

Im erfindungsgemäßen Verfahren wird der Produktionsfermenter nur bei einem Erstansatz oder nach einer Infektion sterilisiert; in den Folgeansätzen erfolgt nur noch die Sterilisation eines Vorlagebehälters. Gegebenenfalls kann das Medium heiß in den Fermenter transferiert und dort durch Wasserdirekteinspritzung schnell herabgekühlt werden.

Die Erfindung betrifft ferner die Verwendung einer Vorrichtung zur Durchführung des erfindungsgemäßen Verfahrens mit einem sterilisierbaren Fermentationskessel, wenigstens einem Tank zur Aufnahme und thermische Sterilisation einer nicht steril filtrierbaren Mediumkomponente und/oder wenigstens einem Vorratsbehälter zur Aufnahme einer steril filtrierbaren Medienkomponente mit einem Sterilfilter zur kontinuierlichen Erzeugung einer sterilen Mediumkomponente sowie einem mit dem Fermentierungskessel verbundenen sterilisierbaren Kreislauf mit Einrichtungen zur Abtrennung von verbrauchtem Nährmedium und Stoffwechselprodukten, bei der der sterilisierbare Kreislauf eine Zentrifuge aufweist und zwischen Fermentationskessel und Zentrifuge eine Haltestrecke vorgesehen ist, deren länge ausreicht, die im Nährmedium noch enthaltenen Nährstoffe aufzubrauchen. Bevorzugte Ausführungsformen di ser Vorrichtung sind Gegenstand der Unteransprüche.

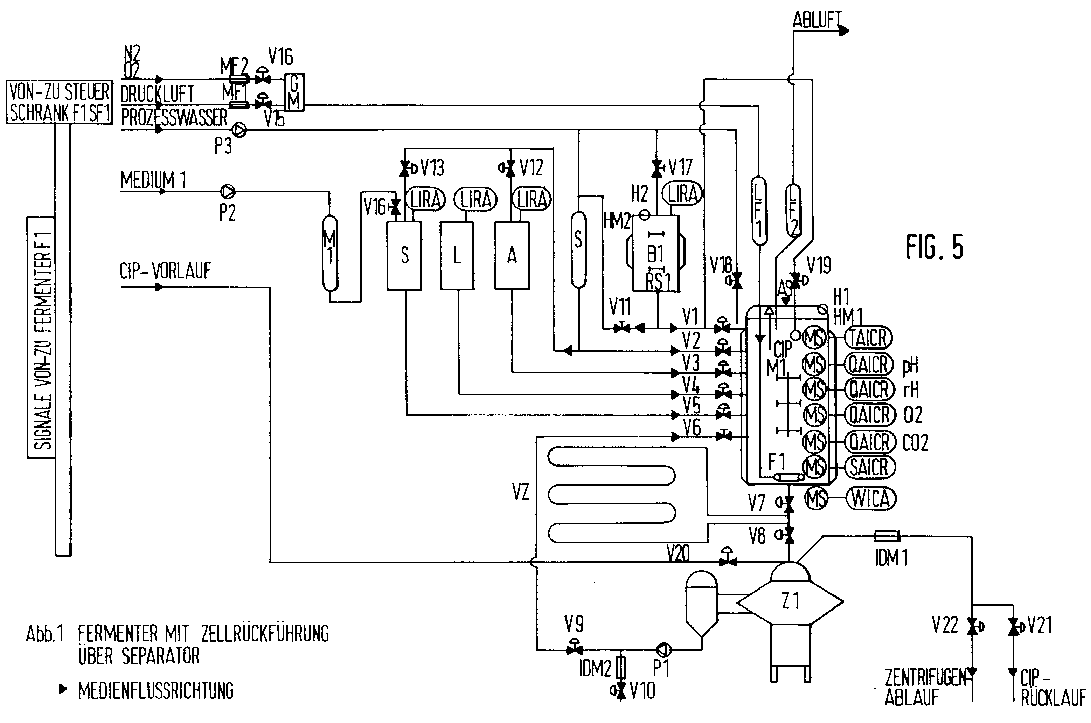

Die erfindungsgemäße Vorrichtung und ihr Betrieb werden nachfolgend mit Bezug auf Fig.5 beschrieben.

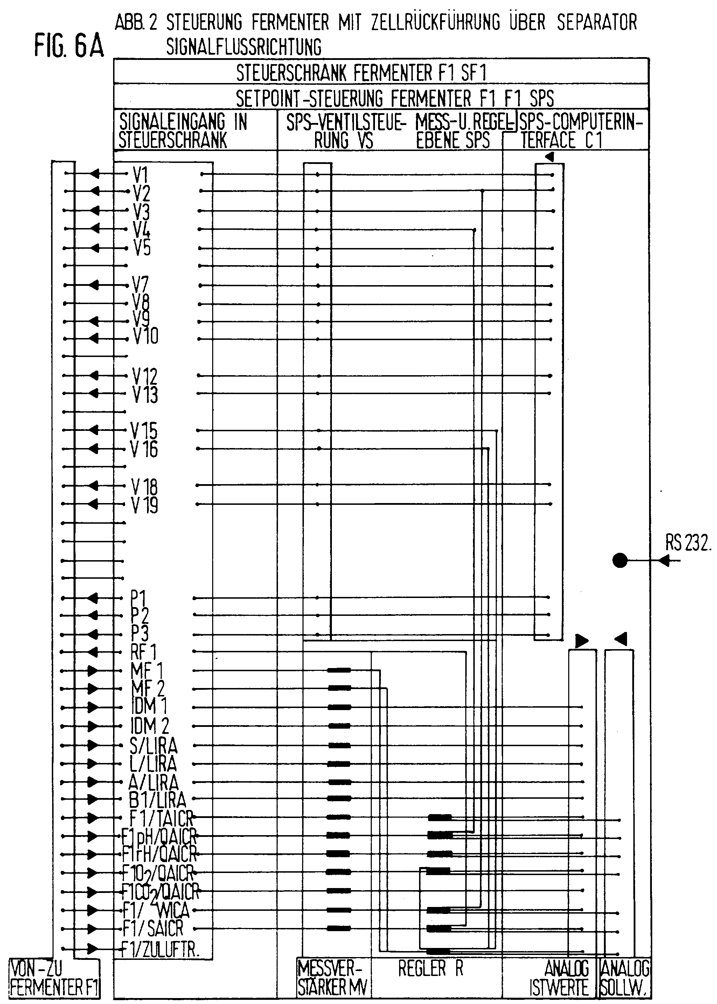

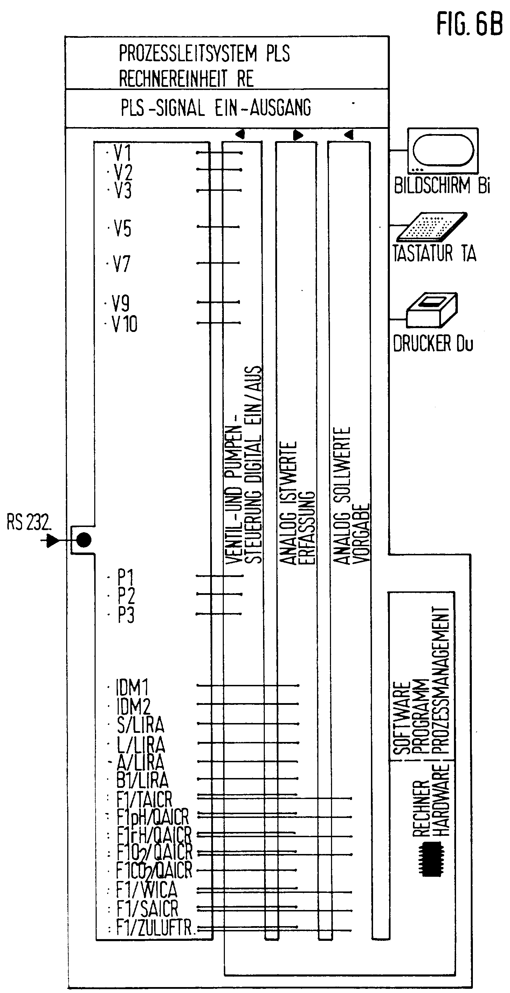

Der Fermenter F1 wird über die Sterilfiltration Luft LF1 belüftet und ist über einen dampfsterilisierbaren Kreislauf mit der Zentrifuge Z1 gekoppelt. Pumpe P1 fördert das in Z1 abgetrennte Konzentrat in F1 zurück. Der Fermenter F1 wird wahlweise über den Tank BI und/oder über die Sterilfiltration S1 mit steril filtriertem oder direkt über den Tank B1 mit thermisch sterilisiertem Medium beschickt. Zugleich können die Mediumsvorlagen S und A mit sterilfiltriertem Medium beschickt werden. Die Mediumvorlage S kann zusätzlich über die Sterilfiltration M1 mit sterilfiltriertem Medium beschickt werden. Das Messen, Steuern und Regeln der gesamten Anlage erfolgt über ein integriertes Prozeßleitsystem, das über die Software prozeßspezifisch programmiert ist und schematisch in Fig.6 dargestellt ist.

Das erfindungsgemäße Verfahren läßt sich in die zuvor aufgeführten sechs Schritte gliedern, die nachfolgend beschrieben sind. Die Erläuterung von Schritt 1 bis 6 erfolgt anhand von Fig.5.

Schritt 1 umfaßt die Vorbereitung und Sterilisation der Fermentationsanlage und der Nährmediumskomponenten und besteht aus sechs Teilschritten.

1.1 Vorlegen von Prozeßwasser in Behälter F1 und Befüllen von Behälter F1 und B1 mit Medium.
1.2 Thermische Sterilisation von nicht sterilfiltrierbarem Mediumkonzentrat in F1.
1.3 Direkteinspritzung von sterilem Kühlwasser in F1 aus der Sterilfiltration S1.
1.4 Transfer von sterilfiltriertem Medium aus Behälter B1 in Fermenter F1 und den Mediumsvorlagen S und A.
1.5 Einstellen des Fermentationsvolumens in F1 mit sterilem Wasser aus S1.
1.6 Erneutes Befüllen von B1 mit Konzentrat aus nicht sterilfiltrierbaren Medienbestandteilen und thermische Sterilisation von B1 mit anschließender indirekter Wasserkühlung.

In Schritt 1.1 wird in Fermenter F1 und Behälter B1 über die Hauptventile Prozeßwasserzulauf V17, V18 eine für die Lösung der Medien notwendige Prozeßwassermenge vorgelegt. Die beiden Behälter werden über die Handlöcher H1, H2 mit Medium befüllt und das Medium klumpenfrei mit den Rührwerken RF1 und RB1 verrührt. In beiden Behältern befinden sich nun Mediumkonzentrate.

Das Mediumkonzentrat in F1 wird in Schritt 1.2 über den Heizmantel HM1 thermisch sterilisiert und auf unter 100°C abgekühlt.

Über Ventil V2 wird dann in Schritt 1.3 steriles Prozeßwasser unter Rühren aus der Sterilfiltration S1 eingespritzt, bis eine Mischtemperatur von ca. 60°C erreicht ist.

Dann wird über Ventil V11 am Behälter B1 über die Sterilfiltration S1 steriles Medium in den Fermenter F1 transferiert. Zugleich wird in Schritt 1.4 sterilfiltriertes Medium über die Ventile V12, V13 in die Mediumvorlagen S, A gegeben.

Im anschließenden Schritt 1.5 wird das Anfangs-Fermentervolumen F1 mit sterilem Prozeßwasser aus der Sterilfiltration S1 über Ventil V2 aufgefüllt. Das Fermenter-Füllvolumen beträgt jetzt ca. 1/3 bis 1/4 des maximalen Arbeitsvolumens. Die Mediumskonzentration ist für den Prozeßbeginn eingestellt, die Mischtemperatur liegt zwischen 30 bis 35°C und damit in der Regel nahe bei der Prozeßtemperatur.

Der leere und gespülte Behälter B1 wird in Schritt 1.6 nach Vorlegen von Prozeßwasser über Ventil V17 über Handloch H2 mit nicht sterilfiltrierbaren Medienbestandteilen gefüllt, diese als Konzentrat über den Heizmantel HM2 thermisch sterilisiert und mittels indirekter Wasserkühlung gekühlt. Die in Behälter B1 und Mediumvorlagen S, A vorliegenden Mediumbestandteile dienen in den weiteren Schritten zur Mediumdosierung. Schritt 2 beinhaltet den ersten Arbeitszyklus der Anlage in ansatzweiser Verfahrensweise mit niederer Medienkonzentration und Teilfüllung und besteht aus vier Hauptschritten.

2.1 Aktivierung des Prozeßleitsystems PLS
2.2 Inokulation des Fermenters F1
2.3 Zellwachstum
2.4 Umschaltung auf Schritt 3 anhand vorgegebener Schaltkriterien.

Das Prozeßleitsystem PLS überwacht und kontrolliert den gesamten Prozeß. Es besteht aus der Rechnereinheit RE, der Tastatur TA, dem Bildschirm Bi und dem Drucker Du. Das Prozeßleitsystem ist über die Datenschnittstelle RS 232 bidirektional mit dem Computerinterface CI der Setpoint-Steuerung Fermenter F1SPS verbunden und steuert über die Ventilsteuerung SPS VS direkt oder indirekt über Sollwertvorgaben an die Regler R, die Ventile V und die Pumpen P an. Alle im Prozeß gemessenen Werte werden über die Meßverstärker MV, das Computerinterface CI, über die Datenschnittstelle RS232 an das Prozeßleitsystem PLS übertragen. Vor Prozeßbeginn wird in Schritt 2.1 die prozeßspezifische Software geladen. Sie beinhaltet alle Alarmwerte, Sollwertvorgaben, Auswertungsfunktionen, Daten zur Ventilsteuerung und die Dokumentation. Die Startbedingungen für den Prozeß werden vom PLS aus vorgegeben und beinhalten die Einstellung beispielsweise folgender Parameter für F1:

Prozeßtemperatur
pH-Wert
Redoxpotential
Sauerstoffpartialdruck
Gewicht
Rührwerkdrehzahl.

Die angesteuerten Ventile V1, V2, V3, V5 und V7 sind geschlossen; die Anlage ist bereit für die Inokulation.

Über den Anstechstutzen AS wird die vorbereitete Animpfkultur in den Fermenter F1 inokuliert und der Prozeß am Prozeßleitsystem PLS gestartet, d.h. die Prozeßleit- und Überwachungsfunktion wird aktiviert.

In der Anfangsphase des Prozesses (Schritt 2.3) läuft das Zellwachstum gemäß einem ansatzweisen Verfahren ab. Der Vorteil beim erfindungsgemäßen Verfahren liegt in der geringen Mediumkonzentration, die ein optimales Anwachsen der Zellen ermöglicht und eine gute Anpassung der Zellen an ihr Medium bewirkt.

Am Ende der ansatzweisen Wachstumsphase, vor Erreichen einer Substratlimitierung, muß über ein Schaltkriterium auf den nächsten Prozeßschritt, die Mediumdosierung, umgeschaltet werden (Schritt 2.4). Die Ermittlung der Schaltkriterien wird nachstehend anhand einiger Beispiele beschrieben.

Als Beispiel für die Bildung eines Schaltkriteriums über stöchiometrische Titration von Stoffwechselproduktion dient ein homofermentativer Lactobacillenstamm, der Glucose nahezu quantitativ in Milchsäure umsetzt.

Bei der Züchtung von Lactobacillen wird der Fermenter F1 pH-statisch gefahren, d.h. der absinkende pH-Wert, bedingt durch die entstehende Milchsäure, wird durch Titration mit Lauge einer definierten Normalität konstant gehalten. Der pH-Wert im Fermenter F1 wird mittels der pH-Regeleinrichtung pH QAICR, bestehend aus Meßsonde MS, Meßverstärker MV und Regler R gemessen und geregelt. Die Meßwerte und Sollwertvorgaben werden mit dem Prozeßleitsystem PLS ausgetauscht, wie oben beschrieben. Die Lauge befindet sich in der Mediumvorlage L und wird über den pH-Regler F1 pH QAICR und das Ventil V4 im Fermenter F1 dosiert. Der Laugenverbrauch in der Mediumvorlage L wird über eine kapazitive Füllstandmeßsonde LIRA gemessen und die Werte im Prozeßleitsystem PLS verarbeitet.

Die Schaltschwelle wird als totale Laugenverbrauchsmenge definiert. Die Ermittlung der Schaltschwelle erfolgt in Vorversuchen, in denen die Zellen im Züchtungsmedium bis in die stationäre Phase gezüchtet werden, der Laugenverbrauch ermittelt wird und dann eine Laugenverbrauchsmenge berechnet wird, die einer Restsubstratmenge (Gluccsemenge) entspricht, bei der die Zellen noch nicht substratlimitiert sind und sich in der exponentiellen Wachstumsphase befinden. Die berechnete Laugenverbrauchsmenge ist somit geringer als die tatsächliche.

Als Beispiel für die Bildung eines Schaltkriteriums auf Basis des Luftbedarfs dient ein Mikrococcenstamm (aerober Stamm). Der Sauerstoffpartialdruck wird während des Wachstums in Schritt 2 auf einen konstanten Wert geregelt. Die $O_2$-Regeleinrichtung $O_2$ QAICR besteht aus Meßsonde MS, Meßverstärker MV und Regler R. Die Meßwerte und Sollwertvorgaben werden gemäß der Beschreibung unter 2.1 mit dem Prozeßleitsystem PLS ausgetauscht. Der Ausgang des Reglers R liegt auf den Regelventilen V15 und V16, die die in den Prozeß eingespeiste Gasmenge steuern.

Während des ansatzweisen Wachstums verbrauchen die Zellen zunehmend mehr Luft, gemessen mit Massendurchflußmengenmesser MF1 und MF2.

Dies gilt, solange Substrat vorhanden ist und die Zellen sich vermehren. Kommen die Zellen in eine Substratlimitierung, sinkt der Luftbedarf bei Konstanthaltung des Sauerstoffpartialdrucks geregelt über F1-$O_2$-QAICR und konstanter Drehzahl (geregelt über SAICR) ab. Dieses Absinken wird als Schaltschwelle genutzt.

Als Beispiel für die Bildung eines Schaltkriteriums auf Basis des $CO_2$-Gehaltes in der flüssigen oder der Gasphase dient ein Leuconostoc-Stamm.

Während des ansatzweisen Wachstums produzieren die Zellen $CO_2$, das sich im Medium löst und über die $CO_2$-Meßeinrichtung F1-$CO_2$-QAIC, bestehend aus Meßsonde MS und Verstärker MV, erfaßt wird. Um Veränderungen in der $CO_2$-Konzentration schneller erkennen zu können, wird der Fermenter F1 über den Massendurchflußmengenmesser $MF_2$ mit Stickstoff begast und mit konstanter Drehzahl gerührt, um $CO_2$ auszutreiben. Kommen die Zellen in eine Substratlimitierung, wird weniger $CO_2$ produziert, der $CO_2$-Partialdruck im Medium sinkt ab. Dieses Absinken wird als Schaltschwelle genutzt.

In Schritt 3 erfolgt der Übergang auf geregelte oder gesteuerte Mediumdosierung bis zum Erreichen des Fermentationsvolumens; er besteht aus zwei Hauptschritten:

3.1 Mediumdosierung
3.2 Umschaltung auf Schritt 4 anhand vorgegebener Schaltkkriterien.

Sobald das Schaltkriterium dafür erfüllt ist, beginnt die geregelte Mediumdosierung. Hierzu wird bei der Steuerung über den pH-Wert der Laugenverbrauch der vergangenen 2 bis 10 Minuten über die Niveaumessung LIRA in Mediumvorlage L ermittelt und vom Prozeßleitsystem in die Dosagemenge an Leitsubstrat (z.B. Glucose) umgerechnet. Dies kann anhand folgender Formel geschehen.

$$SD_{Bed.Gluc} = \frac{k_1 \times k_3 \times 0,5 \times Z \times a \times 180 \text{ g/mol}}{k_2 \times t_1}$$

worin

Z =      Laugenverbrauch in 1
a =      Normalität Lauge (mol)
$t_1$ =      Meßzeit für Laugenverbrauch (min)
$k_1$ =      Korrekturfaktor für die beim Züchtungs-pH-Wert undissoziierte Säure
$k_2$ =      Substratverwertungsfaktor der spez. Kultur
$k_3$ =      Vordosagefaktor
$SD_{Bed.Gluc.}$      Glucosedosagemenge in (g/min).

Das Leitsubstrat steht in einem in Vorversuchen ermittelten, definierten Verhältnis zu den übrigen Substratbestandteilen. Das Prozeßleitsystem errechnet die Dosagemengen für die übrigen Substratkonzentrate und die Wassermenge zur Verdünnung auf Einsatzkonzentration.

Die Mediumkonzentratdosage erfolgt alle 2 bis 5 Minuten. Die sterilfiltrierbaren Mediumbestandteile werden aus dem Mediumtank S über Ventil V5, die thermisch sterilisierbaren Medienbestandteile aus Behälter B1 über Ventil V1 und das Verdünnungswasser über Sterilfiltration S1 über Ventil V2 zudosiert. Die dosierten Mediumsvolumen aus Mediumtank S und Behälter B1 werden über kapazitive Füllstandsmeßsonden LIRA erfaßt. Die Dosage des Verdünnungswassers über Sterilfiltration S1 wird über den Gewichtsregler WICA gesteuert, indem vom Prozeßleitsystem PLS ein steigendes Sollgewicht Fermenter F1 vorgegeben wird.

Mit steigender Zellmasse im System steigt der Verbrauch an Leitsubstrat und somit am Gesamtmedium. Da die Mediumkonzentration konstant gehalten wird, erhöht sich die pro Dosagezyklus dosierte Mediummenge ständig bis zum Erreichen der maximalen Fermenterfüllung. Die Konzentration des dosierten Mediums ist so gewählt, daß bei Erreichen der maximalen Fermenterfüllung die Gesamtmediummenge für die maximale Zellausbeute unter den gegebenen Betriebsbedingungen erreicht ist. Dieser Wert ist die maximale oder spezifische Zellausbeute.

Bei Organismen, bei deren Züchtung kein exakt bestimmbares Leitsubstrat vorliegt, erfolgt die Mediumdosage gesteuert über eine Hüllkurve, die der für den Organismus spezifischen Exponentialfunktion für das Wachstum entspricht. In Vorversuchen wird der spezifische Substratbedarf für 1 x 10$^{12}$ Zellen, die spezifische Wachstumsrate $\mu$, die Keimzahl, die in Schritt 2 zum Zeitpunkt des Schaltkriteriums erreicht wird und die Mediumkonzentration bei der maximal erreichbaren Keimzahl bestimmt.

Sobald das Schaltkriterium, beschrieben unter Schritt 2.4, erfüllt ist, beginnt die gesteuerte Mediumdosierung. Die Mediummenge, die zum Erreichen einer Keimzahl von 1 x 10$^{12}$ Keimen benötigt wird, wird als Mediummenge 1 definiert und ist stammspezifisch.Die Mediumkonzentration des zu dosierenden Mediums wird konstant gehalten und ist so eingestellt, daß bei Erreichen der maximalen Fermenterfüllung die Gesamtmediummenge für die maximale Zellausbeute unter den gegebenen Betriebsbedingungen erreicht ist. Im Verlauf der Züchtung steigen die pro Dosagezyklus dosierten Substratvolumina an.

Die Substratdosage SD berechnet sich nach den Formeln:

$$x_t = e^{\mu \times t + \ln x o} \qquad\qquad (3.1.2.a)$$

$$SD = sS \times x_t \qquad\qquad (3.1.2.b)$$

$$SD_{Ber} = sS \times e^{\mu t + \ln x o} \qquad\qquad (3.1.2.c)$$

$X_o$ = Gesamtkeimzahl im System zu Beginn der Dosage
$X_t$ = Gesamtkeimzahl im System zum Zeitpunkt t
$\mu_o$ = Spezifische Wachstumsrate zu Beginn der Dosage
t = zwischen Messung $x_o$ und $x_t$
sS = Spezifischer Substratbedarf für 1 x 10$^{12}$ Keime in g bzw. ml
$SD_{Ber}$ = Substratdosage berechnet

Die Berechnung der Dosagewerte und Dosierung erfolgt alle 5 bis 15 Minuten. Sind die Dosagevolumina zu Beginn der Dosierung zu klein, werden sie zum nächsten Dosagezyklus addiert und nach Überschreiten in einer minimalen Dosagemenge dosiert.

Steigt bei konstanter Drehzahl des Rührwerks RF1 der Sauerstoffbedarf der Zellen gemessen über Massendurchflußmengenmesser MF1 und MF2 eine bestimmte Zeit nicht mehr an oder fällt ab, was durch eine Substratlimitierung verursacht ist, dann wird die Dosagemenge erhöht, was die Substratlimitierung aufhebt. Ist die Limitierung nach einmaliger Erhöhung von $\mu$ noch nicht aufgehoben, wird nach einer prozeßspezifischen Wartezeit $\mu$ nochmals erhöht.

Die Anpassung der Steuerfunktion an den Prozeß kann nicht nur über den $O_2$-Partialdruck, sondern auch über den $CO_2$-Partialdruck, bestimmt über die $CO_2$-Messung $CO_2$QAICR, erfolgen. Das Meßprinzip ist unter Schritt 2.4.3 beschrieben. Steigt bei konstanter Drehzahl des Rührwerks RF1 und konstanter in den Fermenter F1 eingeblasenen Stickstoffmenge, gemessen über Massendurchflußmengenmesser $MF_2$, der $CO_2$-Partialdruck nicht mehr an oder fällt ab, was durch eine Substratlimitierunq verursacht ist, dann erhöht die Software Prozeßleitsystem PLS die Dosagemenge um einen Betrag, der zu einer Beschleunigung der Nährmediumdosierung führt und die Substratlimitierung aufhebt. Ist die Limitierung nach einmaliger Erhöhung von $\mu$ noch nicht aufgehoben, wird über die Software Prozeßleitsystem PLS nach einer prozeßspezifischen Wartezeit nochmals erhöht.

Das Schaltkriterium von Schritt 3 auf Schritt 4 wird über das Gewicht des Fermenters F1, gemessen mittels Waage F1 WICA, bestimmt. Die Gewichtsschwelle wird prozeßspezifisch gewählt.

Schritt 4 beinhaltet den Übergang auf kontinuierliche Verfahrensweisen mit Nährmediumtausch und vollständiger Zellrückführung oder teilweiser Zellabtrennung und besteht aus vier Hauptschritten.

4.1 Anfahren der Zentrifuge und Beginn der Separation mit Zellrückführung,
4.2 Verfahrensweise mit vollständiger Zellrückführung
4.2.1 Substratdosage während Zellrückführung, alternativ
4.3 Verfahrensweise mit teilweiser Zellabtrennung,
4.3.1 Prozeßführung mit vollständiger Zellrückführung bis Beginn Teilabtrennung,
4.3.2 Umschaltung auf teilweise Zellabtrennung,
4.3.3 Teilabtrennung,

sowie

4.4 Abbruch und Umschaltung auf Schritt 5.

Vor Erreichen der Schaltschwelle gemäß Schritt 3.2 wird der Separator Z1 eingeschaltet und nach Erreichen der Schaltschwelle das Ventil V7 über das Prozeßleitsystem PLS geöffnet. Über Ventil V8 wird bereits vor der Separierung die maximale verfahrensspezifische Durchflußrate des Separators eingestellt. Die eingestellte Durchflußrate wird vom induktiven Durchflußmengenmesser IDM 1 aufgenommen und über das Prozeßleitsystem PLS erfaßt. Zur Rückführung der abgetrennten Zellmasse wird parallel zum Ventil V7 die Pumpe P1 geschaltet. Die Ventile V9 und V6 sind von Prozeßbeginn an geöffnet.

Die Konsistenz der abgetrennten Zellmasse muß flüssig sein, damit die Rückführung der Zellmasse gemäß Schritt 4.2 über Pumpe P1 möglich ist und die Rückvermischung der Zellmasse im Fermenter in kurzer Zeit erfolgt. Bei Sedimentanteilen < 60 % entspricht die Konsistenz in der Regel diesen Anforderungen. Der Eindickungsgrad darf nicht unterhalb 40% Sediment fallen, da dann das rückgeführte Mediumvolumen die effektive Trennleistung des Separators Z1 bis zu 15% herabsetzt.

Der Eindickungsgrad wird über die Entleerzeit des Separators Z1 gesteuert. Die Entleerung erfolgt etwa alle 3 bis 4 Minuten, da die Verweilzeit der Zellen im Separator nicht über diese Zeitspanne ansteigen darf. Dies ergibt in Abhängigkeit von der verwendeten Zellart die geringste Zellschädigung.

Die Substratdosage erfolgt während der Zellrückführung in Abhängigkeit von den gewählten Parametern weiter oder wird gegebenenfalls für Zeit der Zellrückführung eingestellt. Zellrückführung ohne Substratdosage ist bei denjenigen Organismen notwendig, die erst in vollständig glucosefreiem Medium gute Sedimentationseigenschaften aufweisen (s. Beispiel 1). Für Organismen, bei deren Züchtung die Substratdosage während der Zellrückführung erfolgt, kann dies beispielsweise wie folgt geschehen.

Bei der Dosage über die Erfassung eines Leitsubstrates erfolgt die Substratzugabe bedarfsorientiert. Die Dosierung des Verdünnungswassers erfolgt über Sterilfiltration S1 und Ventil V2, gesteuert durch die Gewichtsregelung WICA des Fermenters F1, die das Fermentergewicht unabhängig von der aktuellen Trennleistung der Zentrifuge Z1 konstant hält. Der Gewichtssollwert ist vorgegeben und entspricht dem prozeßspezifischen Arbeitsvolumen im Fermenter F1. Die aus Mediumtank S über Ventil V5 und Behälter B1 über Ventil V1 dosierten Mediumkonzentratmengen werden über den Verbrauch an Leitsubstrat ermittelt und dosiert. Der Verlauf der in den Prozeß eingeführten Gesamtsubstratmenge und der zeitbezogenen Substratdosiermengen stellt eine Exponentialfunktion dar bis zum oberen Grenzpunkt, ab dem die Dosierung nicht mehr bedarfsorientiert, sondern linear erfolgt. Der obere Grenzpunkt ergibt sich aus der maximal möglichen Mediumkonzentration im Zulauf, die einen in Vorversuchen ermittelten Wert nicht übersteigen darf.

Diese maximale Zulaufmenge orientiert sich an der aktuellen effektiven Trennleistung Teff der Zentrifuge. Die bedarfsorientierte Substratdosierung ist immer kleiner oder gleich der aus Teff berechneten maximalen Substratdosierung.

Im Vergleich zu einem herkömmlichen ansatzweisen Verfahren ist die durch diese Steuerung im erfindungsgemäßen Verfahren erreichte Keimzahl um wenigstens den Faktor 10 höher.

Die Dosage kann auch über vorausberechnete Werte (Hüllkurve) analog zur vorstehend beschriebenen Dosierung über die Erfassung eines Leitsubstrates erfolgen.

Die Verfahrensweise mit teilweiser Zellabtrennung (Schritt 4.3) entspricht zu Beginn dem unter 4.2 beschriebenen Verfahren mit vollständiger Zellrückführung. Der Prozeß wird unter vollständiger Zellrückführung an den Arbeitspunkt für den Beginn der teilweisen Zellabtrennung herangeführt.

Das Schaltkriterium für den Beginn der teilweisen Zellabtrennung ist prozeßspezifisch, ist jedoch immer mit der spezifischen Wachstumsrate $\mu$ gekoppelt, da Substratverwertung und Produktion von Stoffwechselprodukten direkt von der spezifischen Wachstumsrate abhängen. $\mu$ ist organismusspezifisch und wird in Vorversuchen festgelegt.

Aus der ins Gesamtsystem eingetragenen Substratmenge wird über den spezifischen Substratbedarf pro Zellzahl die Gesamtkeimzahl im System errechnet. Diese entspricht bei definiertem Systemvolumen definierter maxim. Mediumkonzentration und bei definierter Separationsleistung einer definierten Wachstumsrate $\mu$, die den Schaltpunkt darstellt.

Die zum Zeitpunkt der Teilabtrennung erreichte Keimzahl im System wird durch die Entnahme eines definierten Zellmassenstromes aus dem System konstant gehalten.

Zur Entnahme von Zellmasse aus dem System wird bei laufender Pumpe P1 das Ventil V9 geschlossen und das Ventil V10 geöffnet. Über den Durchflußmengenmesser IDM2 wird die Ablaufmenge an Konzentrat erfaßt und nach Erreichen der berechneten Menge die Ventile V10 geschlossen und V9 geöffnet. Das Zeitintervall für einen Entnahmezyklus liegt bei etwa 3-10 Minuten.

Erfolgt die Substratdosage über ein Leitsubstrat, wird die zum Zeitpunkt des Beginns der Teilabtrennung aktuelle Dosagemenge konstant weiterdosiert. Befindet sich die Dosierung an der oberen Grenze, wird diese beibehalten.

Bei Dosage über vorausberechnete Werte (Hüllkurve) wird ebenfalls die zum Zeitpunkt des Beginns der Teilabtrennung aktuelle Dosagemenge konstant weiterdosiert. Befindet sich die Dosierung an der oberen Grenze, wird diese beibehalten.

Diese Dosage ermöglicht ein Nachwachsen der abgetrennten Zellmasse. Bei Konstanthaltung der Zu- und Ablaufverhältnisse stellt sich im System ein stabiles Gleichgewicht ein. Die Zellen wachsen mit konstanter Wachstumsrate μ.

Das Abbruchkriterium für den kontinuierlichen Schritt wird beim Verfahren mit vollständiger Zellrückführung über den Gesamtsubstrateinsatz gebildet. Mit einer prozeßabhängigen Gesamtsubstratmenge wird die prozeßabhängige Gesamtkeimzahl im System mit einer definierten Wachstumsrate μ gebildet. Ab einer bestimmten Wachstumsrate wird die Substratverwertung als nicht mehr ausreichend definiert.

Das Abbruchkriterium für den kontinuierlichen Schritt wird beim Verfahren mit teilweiser Zellabtrennung durch Systemfehler wie Infektionen, Fouling oder Maschinendefekte gebildet, oder ergibt sich aus wirtschaftlichen oder sonstigen Überlegungen.

Schritt 5 besteht im Abbruch des kontinuierlichen Fermentationsabschnittes und in der Ernte der gesamten Biomasse unter sterilen Bedingungen.

Bei Organismen, deren Sedimentationseigenschaften erst im glucosefreien Medium für die Ernte ausreichend sind, wird ohne Substratdosage bei maximaler Trennleistung der Zentrifuge geerntet.

Die Ernte mit Substratdosierung verläuft für Ansätze mit Substratdosierung über Leitsubstrat oder Hüllkurve identisch und erlaubt eine Zellernte unter Erhaltung der Stoffwechselaktivität. Die Ernte wird durch Öffnen von Ventil V1 und Schließen von Ventil V9 eingeleitet. Für die Ernte vorgegeben wird eine zeitliche Gewichtsabnahme des Fermenters $F1\Delta MF1$, gesteuert über den Gewichtsregler WICA F1, die aus der maximal zulässigen Erntedauer resultiert und kleiner sein muß als die maximale Trennleistung der Zentrifuge, gemessen über IDM1.

Schritt 6 besteht im Neuansatz steriler Nährmedien in die sterile Anlage und Wiederholung des Prozesses gemäß der Schritte 1 bis 5 unter Einsparung der gesamten Sterilisationszeit und Energie (Split-Batch-Betrieb).

Direkt anschließend an die Ernte werden alle Ventile geschlossen. Fermenter F1 wird über Ventil V19 und die CIP-Kugel mit sterilfiltriertem Wasser aus Sterilfiltration S1 gespült, um u.U. vorhandene Foulingansätze (Verschmutzungen) zu beseitigen. Das verschmutzte Wasser wird über die Ventile V7 und V8 in die Zentrifuge Z1 gefahren und dient hier zur Vorspülung.

Nach dem Spülschritt werden die Ventile V19, V7, V8 geschlossen.

Die Zentrifuge Z1 wird nun über die Ventile V20, V21 CIP (cleaning in place) gereinigt. Dies ist notwendig, um in der Zentrifuge vorhandene Zellmassenrückstände zu beseitigen, da diese bis zum Beginn des folgenden Separationszyklus in Lysis übergehen würden. Nach der Reinigung werden die Ventile V20, V21 geschlossen und die Zentrifuge über die Hauptventile V7, V8, V10, V22 dampfsterilisiert. Der Zeitpunkt der Sterilisation wird in den unter Schritt 2 beschriebenen ansatzweisen Verfahrensschritt gelegt. In diesem Schritt wird der Separator nicht gebraucht.

Die Nährmedien für den folgenden Prozeßzyklus werden im Behälter B1 vorbereitet. Zuerst werden im Behälter B1 die sterilfiltrierbaren Medienbestandteile hergestellt und diese analog zu Schritt 1.4 über Ventile V11 aus Behälter B1 über die Sterilfiltration S1 und Ventil V2 manuell in Fermenter F1 transferiert. Zugleich wird sterilfiltriertes Medium über die manuell betätigten Ventile V12, V13 in die Mediumvorlagen S, A gegeben.

Anschließend wird Behälter B1 nach Vorlegen von Prozeßwasser über Ventil V17 mit nicht sterilfiltrierbaren Medienbestandteilen gefüllt, diese als Konzentrat über den Heizmantel HM2 thermisch sterilisiert und mittels indirekter Wasserkühlung über HM2 gekühlt. Während der Sterilisation von Behälter B1 wird über die Sterilfiltration S1 und Ventil V2 eine Menge steriles Prozeßwasser auf das sterilfiltrierte Mediumkonzentrat in F1 gegeben, so daß nach Dosage des heißen, thermisch sterilisierten Mediumkonzentrates aus Behälter B1 über Ventil V1 das Fermentationsanfangsvolumen im Fermenter F1 vorliegt.

Der Prozeß verläuft im folgenden wie in den Schritten 2 bis 6 beschrieben.

Beispiel 1

Durchführung einer verfahrenstypischen Fermentation am Organismus Lactobacillus curvatus Stamm 2 DSM Nr. 4264.

Die Durchführung des Verfahrens läßt sich in fünf Schritte gliedern, die nach dem Start der Anlage zyklisch ablaufen.

Schritt 1: Vorbereitung und Sterilisation der Fermenteranlage und der Nährmediumkomponenten.

Vor Beginn einer Verfahrensdurchführung wird die gesamte Anlage auf Dichtigkeit geprüft und anschließend die Startvoraussetzungen für das Ansetzen der Medien durch Stellen der entsprechenden Ventile geschaffen. Anschließend wird die Fermenteranlage vorbereitet. Die Nährmedien werden eingewogen und in die Tanks und Behälter eingefüllt. Die Anlage wird sterilisiert.

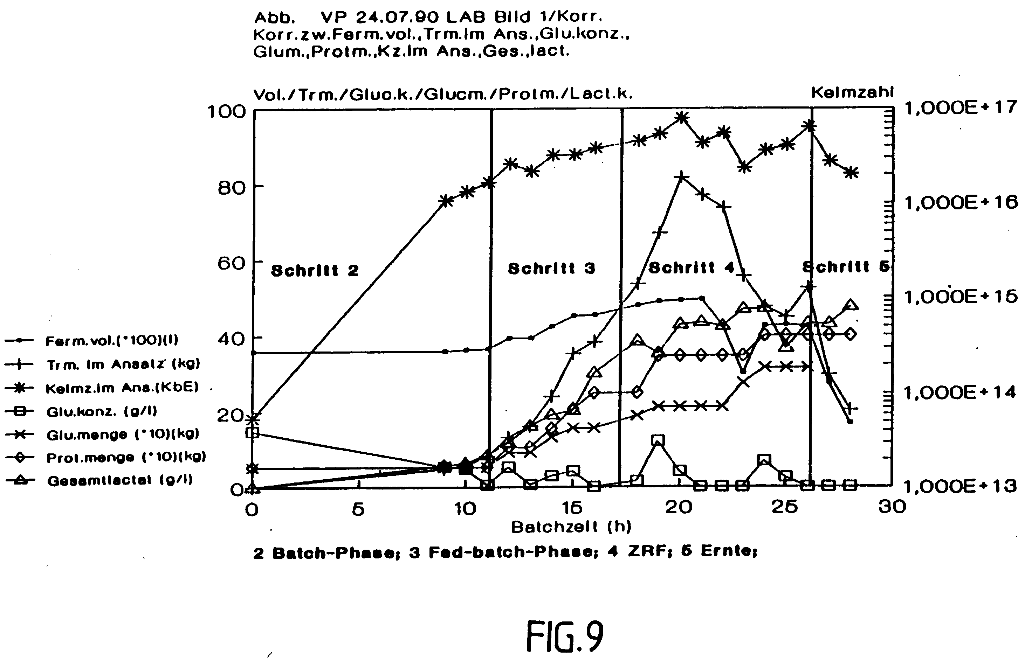

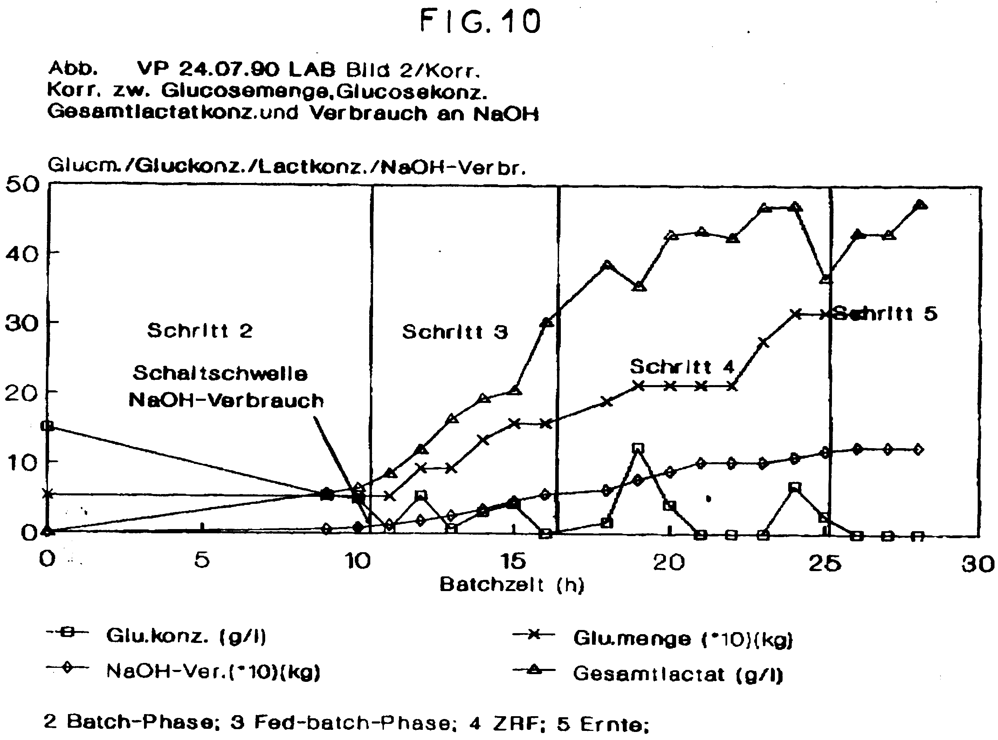

Fig.9 und 10 zeigen den Verlauf der Anlagen- und Prozeßparameter.

Schritt 2: Start der Fermentation durch Animpfen des Fermenters mit Animpfkultur, Start des Prozeßleitsystems und erster Arbeitszyklus der Anlage in ansatzweiser Verfahrensweise mit niederer Mediumkonzentration und Teilfüllung.

Während des Verlaufs werden alle Betriebsparameter konstant gehalten. Am Ende von Schritt 2 wird der Schaltpunkt auf Schritt 3 erreicht. Der Schaltpunkt ist eine definierte Glucosekonzentration im Medium. Bis zum Erreichen der als Schaltschwelle gesetzten Glucosekonzentration von ca. 1 g/l Glucose wird vom Beginn der Fermentation an eine definierte Menge an Natronlauge verbraucht. Ist dieser Verbrauch erreicht, wird geschaltet.

Schritt 3: Übergang auf geregelte Mediumdosierung bis zum Erreichen des Fermentationsendvolumens.

Das Medium wird über die Leitsubstanz Natronlauge geregelt zudosiert. Im Beispiel wird die Dosage in einer Treppenfunktion durchgeführt, was beim vorliegenden Mikroorganismus sehr schnelles Wachstum induziert (s. Fig.10).

Bei anderen Mikroorganismen ist eine stetige Dosage für optimales Wachstum notwendig. Über das Prozeßleitsystem lassen sich beliebige Dosagefunktionen fahren.

Schritt 4: Übergang auf kontinuierliche Verfahrensweise mit Nährmediumtausch und vollständiger Zellrückführung oder mit teilweiser Zellabtrennung.

Beim vorliegenden Organismus wird zu Beginn von Schritt 4 das Fermentervolumen unter vollständiger Zellrückführung eingeengt und anschließend Medium zudosiert. Im weiteren Verlauf des Verfahrens findet eine weitere Zellrückführung mit teilweiser Zellabtrennung statt. Anschließend wird wieder Medium dosiert. Der beschriebene Ablauf des Schrittes 4 ist charakteristisch für den verwendeten Organismus. Andere Organismen werden mit veränderten Trenn- und Dosageintervallen oder mit stetiger Trennung und Dosage gefahren (s. Fig.9).

Schritt 5: Abbruch des kontinuierlichen Fermentationsabschnittes und Ernte der gesamten Biomasse unter sterilen Bedingungen.

Nach dem vollständigen Verbrauch der Nährmedien, ersichtlich am Ende des Natronlaugeverbrauchs, wird die Ernte eingeleitet. Die Ernte wird mit maximaler Trennleistung der Zentrifuge ohne Zellrückführung gefahren. Bedingt durch den verwendeten Organismus ist die Separatorleistung, verglichen mit der bei anderen Organismen, klein. Die kleine Trennleistung ist auf die den Organismus umgebende Saccharidhülle zurückzuführen, die eine Sedimentation erschwert. Andere Organismen ohne Saccharidhülle ermöglichen 2- bis 3-fach höhere Trennleistungen des Separators. Der im Beispiel behandelte Organismus stellt aufgrund der o.g. Saccharidhüllenbildung und des dadurch bedingten schlechten Sedimentationsverhaltens hohe Anforderungen an den Verfahrensablauf, da nur relativ kleine Medientauschraten gefahren werden können.

Schritt 6: Neuansatz steriler Nährmedien in die sterile Anlage und Wiederholung des Prozesses gemäß der Schritte 1-5 unter Einsparung der gesamten Sterilisationszeit und Energie.

Am Ende des Schrittes 4 wird in den Medientanks eine für den nieder konzentrierten Neuansatz notwendige Mediummenge belassen. Direkt nach Schritt 5 wird steriles Mediumkonzentrat und steriles Wasser in den Fermenter F1 gepumpt und sofort anschließend eine Inokulation des Ansatzes mit einer neuen Animpfkultur durchgeführt. Anschließend, während Schritt 2 des Verfahrens, werden in den Mediumvorlagetanks B1, S-Vorlage und A-Vorlage die Dosagemedien für den laufenden Ansatz vorbereitet.

Das Verfahren wird mit den folgenden Stämmen wiederholt:

| Beispiel 2: | Lactobacillus curvatus Stamm 3 | DSM 4265 |
|---|---|---|
| Beispiel 3: | Pediococcus pentosaceus | DSM 6165 |
| Beispiel 4: | Pediococcus acidilactici | DSM 6164 |
| Beispiel 5: | Staphylococcus carnosus Sc1 | DSM 6162 |
| Beispiel 6: | Micrococcus varians M 28 | DSM 6163 |
| Beispiel 7: | Micrococcus varians M 101 | DSM 4263 |

Die Züchtungsparameter sind in der folgenden Tabelle zusammengefaßt.

| Züchtungsparameter Starterkulturstämme | | | | | | | |
|---|---|---|---|---|---|---|---|
| Stamm | LAB[1]) | LAC[2]) | PEB[3]) | PEC[4]) | SAB[5]) (ScA 1) | MIB[6]) (M 28) | MIC[7]) (M 101) |
| DSM Nr. | 4264 | 4265 | 6165 | 6164 | 6162 | 6163 | 4263 |
| Züchtungstemperatur | 30°C | 30°C | 30°C | 30°C | 35°C | 35°C | 35°C |
| Züchtungs-pH | 5,9 | 5,9 | 5,9 | 5,9 | 6,5 | 6,5 | 6,5 |
| Belüftung %$O_2$ vvm | - | - | - | - | 20% $O_2$ | 20% $O_2$<br>0,14 vvm | 20% $O_2$<br>0,14 vvm |
| Begasung | $N_2$ | $N_2$ | $N_2$ | $N_2$ | Rein$O_2$ | Rein$O_2$ | Rein$O_2$ |
| Rührerdrehzahl | 120 l/min | 120 l/min | 120 l/min | 120 l/min | 80-250 l/min | 80-250 l/min | 80-250 l/min |
| Animpfverhältnis | 1:562 | 1:562 | 1:1125 | 1:563 | 1:1000 | 1:80 | 1:80 |
| Animpfkultur | Kultur in log-Phase überimpfen | | | | Kult.in log-Ph. überimpft | Kultur in log-Phase überimpfen | |
| Züchtungsdauer | 24-26h | 24-26h | 24-26h | 24-30h | 14-17h | 18-20h | 18-20h |
| Laugenverbrauch | 333 g NaOH/1000g Glucose | 333 g NaOH/1000g Glucose | 333 g NaOH/1000g Gluc. | 333 g NaOH/1000g Glucose | 266 g NaOH/1000g Glucose | 24 g NaOH/1000g Glucose | 24 g NaOH/1000g Glucose |
| Erntezeitpunkt | Ende Gluc. | Ende Gluc. | Ende Gluc. | Ende Gluc. | Ende Gluc. | Ende Gluc. | Ende Gluc. |
| Separationsleistung | 1400 l/h | 1400-1600 l/h | 2000-2400 l/h | 2000-2400 l/h | 3200 l/h | 3400 l/h | 3400 l/h |
| Fermenterarbeitsvolumen | 4500 l | 4500 l | 4500 l | 4500 l | 4000 l | 4000 l | 4000 l |
| Schaltschwelle von Schnitt 2 (Batch-Ferm) auf Schnitt 3 (Fed-Batch-Ferm) | 1 g/l Glucose-konzentr. im Medium | 1 g/l Glucose-konzentr. im Medium | 1 g/l Glucose-konzentr. im Medium | 1 g/l Glucose-konzentr. im Medium | 3 g/l Glucose-konzentr. im Medium | Anstieg $O_2$-Partial-druck od. Anstieg Luftbedarf | |

[1]) Lactobacillus curvatus Stamm 2

[2]) Lactobacillus curvatus Stamm 3

[3]) Pediococcus pentosaceus

[4]) Pediococcus acidilactici

[5]) Staphylococcus carnosus Stamm 1

[6]) Micrococcus varians Stamm M28

[7]) Mikrococcus varians Stamm M101

Beispiel 3

Durchführung einer Fermentation am Organismus Pediococcus pentosaceus.

Die Durchführung des erfindungsgemäßen Verfahrens läßt sich in fünf Schritte gliedern, die nach dem Start der Anlage zyklisch ablaufen.

Schritt 1: Vorbereitung und Sterilisation der Fermenteranlage und der Nährmediumkomponenten.

Vor Beginn einer Verfahrensdurchführung wird die gesamte Anlage auf Dichtigkeit geprüft und anschließend die Startvoraussetzungen für das Ansetzen der Medien durch Stellen der entsprechenden Ventile geschaffen. Anschließend wird die Fermenteranlage vorbereitet. Die Nährmedien werden gemäß Rezeptur Pediococcus pentosaceus eingewogen und in die dort angegebenen Tanks und Behälter eingefüllt. Die Anlage wird sterilisiert.

Schritt 2: Start der Fermentation durch Animpfen des Fermenters mit Animpfkultur, Start des Prozeßleitsystems und erster Arbeitszyklus der Anlage in ansatzweiser Verfahrensweise mit niederer Mediumkonzentration und Teilfüllung.

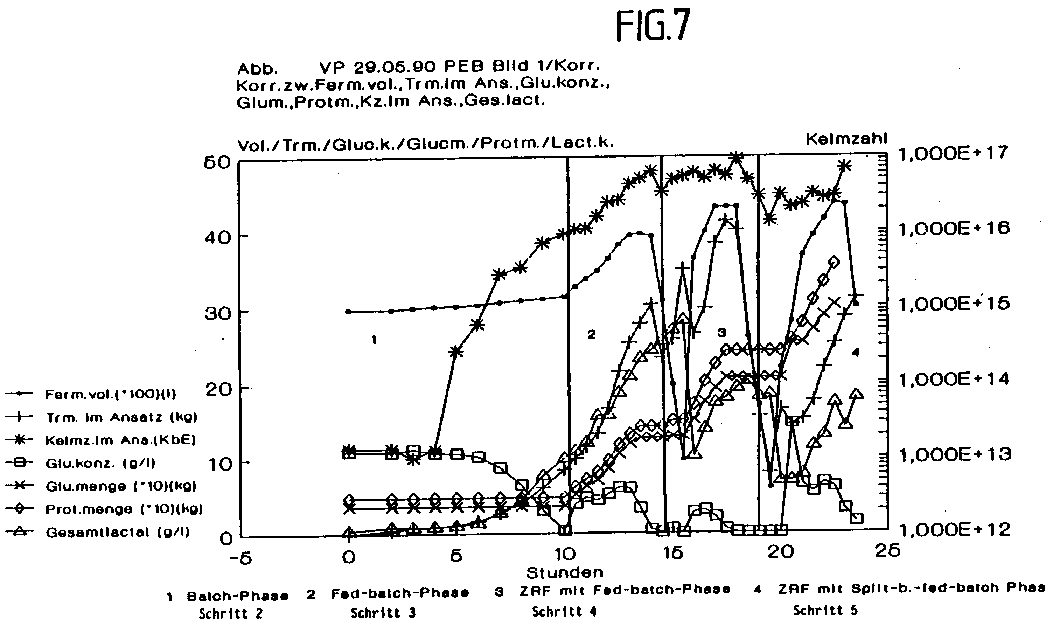

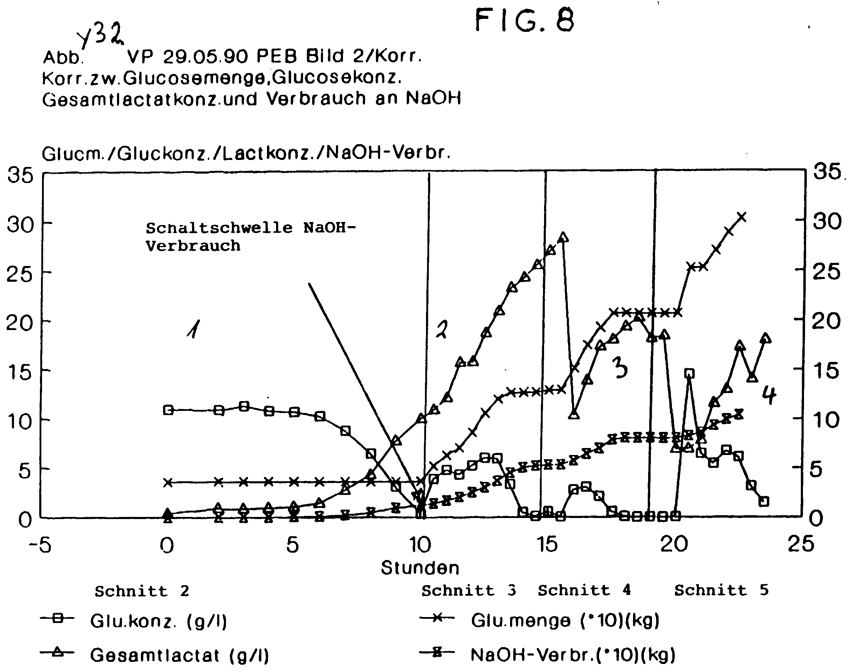

Fig.7 und 8, Bereich 1, Batch Phase zeigen den Verlauf der Anlagenparameter. Alle Betriebsparameter werden konstant gehalten. Am Ende von Schritt 2 wird der Schaltpunkt auf Schritt 3 erreicht. Im vorliegenden Beispiel ist der Schaltpunkt eine definierte Glucosekonzentration im Medium. Bis zum Erreichen der als Schaltschwelle gesetzten Glucosekonzentration von ca. 1 g/l Glucose wird vom Beginn der Fermentation an eine definierte Menge an Natronlauge verbraucht. Ist dieser Verbrauch erreicht, wird geschaltet (s. Fig.8).

Schritt 3: Übergang auf geregelte Mediumdosierung bis zum Erreichen des Fermentationsendvolumens.

Im vorliegenden Beispiel wird das Medium über die Leitsubstanz Natronlauge geregelt zudosiert. Im Beispiel wird die Dosage stetig durchgeführt, was beim vorliegenden Mikroorganismus sehr schnelles Wachstum induziert. Die stetige Dosage ist in Fig.7 am Ansteigen des Fermentervolumens, der Glucosemenge und der Proteinmenge zu sehen. Bei anderen Mikroorganismen ist eine treppenförmige Dosage für optimales Wachstum notwendig. Über das Prozeßleitsystem lassen sich beliebige Dosagefunktionen fahren.

Schritt 4: Übergang auf kontinuierliche Verfahrensweise mit Nährmediumtausch und vollständiger Zellrückführung oder mit teilweiser Zellabtrennung.

Zu Beginn von Schritt 4 wird das Fermentervolumen unter vollständiger Zellrückführung eingeengt und anschließend Medium zudosiert. Im weiteren Verlauf des Verfahrens findet eine weitere Zellrückführung mit teilweiser Zellabtrennung statt. Anschließend wird wieder Medium dosiert. Der beschriebene Ablauf des Schrittes 4 ist charakteristisch für den verwendeten Organismus. Andere Organismen werden mit veränderten Trenn- und Dosageintervallen oder mit stetiger Trennung und Dosage gefahren.

Schritt 5: Abbruch des kontinuierlichen Fermentationsabschnittes und Ernte der gesamten Biomasse unter sterilen Bedingungen.

Nach dem vollständigen Verbrauch der Nährmedien, beim vorliegenden Organismus ersichtlich am Ende des Natronlaugeverbrauchs, wird die Ernte eingeleitet. Die Ernte wird mit maximaler Trennleistung der Zentrifuge ohne Zellrückführung gefahren.

Schritt 6: Neuansatz steriler Nährmedien in die sterile Anlage und Wiederholung des Prozesses gemäß der Schritte 1-5 unter Einsparung der gesamten Sterilisationszeit und Energie.

Am Ende des Schrittes 4 wird in den Medientanks eine für den nieder konzentrierten Neuansatz notwendige Mediummenge belassen. Direkt nach Schritt 5 wird steriles Mediumkonzentrat und steriles Wasser in den Fermenter F1 gepumpt und sofort anschließend eine Inokulation des Ansatzes mit einer neuen Animpfkultur durchgeführt. Anschließend, während Schritt 2 des Verfahrens, werden in den Mediumvorlagetanks B1, S-Vorlage und A-Vorlage die Dosagemedien für den laufenden Ansatz vorbereitet.

**Patentansprüche**

1. Verfahren zur Erzeugung von Zellmasse und/oder Fermentierungsprodukten unter sterilen Bedingungen, bei dem das Fermentierungsgemisch wenigstens zeitweise im Kreislauf geführt wird und Stoffwechselprodukte der kultivierten Zellen und gegebenenfalls Zellmasse abgetrennt werden, welches Verfahren die folgenden Schritte aufweist

   - Beschicken der Fermentationsanlage mit einer zum Starten der gewünschten Zellkultur ausreichenden Menge an Nährmedium;
   - Sterilisieren der Anlage sowie Einstellen der gewünschten Nährmediumkonzentration die niedriger ist, als die spezifische Nährmediumkonzentration der Zellkultur;
   - Beimpfen des Nährmediums mit der Animpfkultur und ungestörtes Wachsenlassen der Kultur über einen definierten Zeitraum in ausatzweiser Verfahrensweise und unter Teilfüllung der Fermentationsaulage;

- Steigerung der Konzentration des Nährmediums auf die spezifische Nährmediumkonzentration der Zellkultur bei gleichzeitiger Steigerung des Nährmediumvolumens auf das Arbeitsvolumen der Anlage sowie der Zellkonzentration;
- Übergang zu kontinuierlicher Verfahrensweise unter Austausch des Nährmediums und Abtrennung der Stoffwechselprodukte sowie vollständiger oder teilweiser Zellrückführung;
- Abbruch der kontinuierlichen Verfahrensweise zu einem gewünschten Zeitpunkt und Ernte der Zellmasse unter sterilen Bedingungen; und gegebenenfalls
- Wiederholung eines Teils der oder aller vorgenannter Schritte in der genannten Reihenfolge

2. Verfahren nach Anspruch 1,
   dadurch **gekennzeichnet,** daß in der Phase des Mediumtausches die zudosierte Mediummenge mit dem exponentiellen Wachstum der Kultur erhöht wird und bei Erreichen der maximalen Zellabtrennung konstant gehalten wird.

3. Verfahren nach Anspruch 1 oder 2,
   dadurch **gekennzeichnet,** daß die Zudosierung des Nährmediums über einen Leitparameter gesteuert wird, dessen Wert ständig bestimmt wird.

4. Verfahren nach Anspruch 3,
   dadurch **gekennzeichnet,** daß als Leitparameter der pH-Wert, die $CO_2$ oder die $O_2$-Konzentration verwandt wird.

5. Verfahren nach Anspruch 4,
   dadurch **gekennzeichnet,** daß in der Fermentationsanlage der pH-Wert bestimmt und durch Zugabe einer Base definierter Konzentration konstant gehalten wird.

6. Verfahren nach Anspruch 5,
   dadurch **gekennzeichnet,** daß eine zur verbrauchten Basenmenge äquivalente Menge Nährmedium zugesetzt wird, die in der Wachstumsphase der Zellkultur um einen zur Wachstumsrate proportionalen Dosagefaktor k verändert wird.

7. Verfahren nach Anspruch 3,
   dadurch **gekennzeichnet,** daß verbrauchtes Nährmedium anhand einer für die Zellkultur spezifischen Hüllkurve, die die vorhandenen Zellmassen, die Wachstumsrate und die Kulturbedingungen berücksichtigt, ersetzt wird.

8. Verfahren nach einem der Ansprüche 1 bis 7,
   dadurch **gekennzeichnet,** daß es kontinuierlich durchgeführt wird.

9. Verfahren nach Anspruch 8,
   dadurch **gekennzeichnet,** daß die Zellkultur durch Variation des Ernteanteils bzw. der Zellrückführung auf eine gewünschte Wachstumsrate eingestellt wird.

10. Verfahren nach einem der Ansprüche 1 bis 9,
    dadurch **gekennzeichnet,** daß die Stoffwechselprodukte und die Zellmasse durch Zentrifugieren abgetrennt werden.

11. Verfahren nach einem der Ansprüche 1 bis 10,
    dadurch **gekennzeichnet,** daß die Fermentationsanlage anfangs mit einem Nährmediumkonzentrat beschickt wird.

12. Verfahren nach Anspruch 11,
    dadurch **gekennzeichnet,** daß das Einstellen der Anfangsnährmedium-Konzentration durch Kalteinspritzung von Sterilwasser erfolgt.

13. Verfahren nach einem der Ansprüche 1 bis 12,
    dadurch **gekennzeichnet,** daß das Nährmedium als Konzentrat zudosiert wird.

14. Verfahren nach einem der Ansprüche 1 bis 13,
    dadurch **gekennzeichnet,** daß in der Erntephase die Zellmasse teilweise zurückgeführt wird.

15. Verfahren nach einem der Ansprüche 1 bis 14,
dadurch **gekennzeichnet,** daß bei der Züchtung aerober Zellen der Zellkultur mit Reinsauerstoff angereicherte Luft zugeführt wird.

16. Anwendung des Verfahrens nach einem der Ansprüche 1 bis 15 auf die Gewinnung von Milchsäurebakterien und/oder Milchsäure.

17. Verwendung einer Vorrichtung zur Erzeugung von Zellmasse und/oder Fermentierungsprodukte mit einem sterilisierbaren Fermentationskessel, wenigstens einem Tank zur Aufnahme und thermische Sterilisation einer nicht sterilfiltrierbaren Mediumkomponente und/oder wenigstens einem Vorratsbehälter einer sterilfiltrierbaren Mediumkomponente mit einem Sterilfilter zur kontinuierlichen Erzeugung einer sterilen Mediumkomponente sowie einem mit dem Fermentierungskessel verbunden sterilisierbarem Kreislauf mit Einrichtungen zur Abtrennung von verbrauchtem Nährmedium und Stoffwechselprodukten, wobei der sterilisierbare Kreislauf eine Zentrifuge aufweist und zwischen Fermentationskessel und Zentrifuge eine Haltestrecke vorgesehen ist, deren Länge ausreicht, die im Nährmedium noch enthaltenen Nährstoffe aufzubrauchen, zur Durchführung des Verfahrens nach Anspruch 1.

18. Verwendung nach Anspruch 17, daurch gekennzeichnet, daß die Zentrifuge eine dampfsterilisierbare, sich selbst entschlammende Zentrifuge mit einstellbaren Entschlammungsmengen ist.

19. Verwendung nach Anspruch 17 oder 18, dadurch gekennzeichnet, daß im Fermentationskessel eine Meßsonde zur Erfassung einer Prozeßsteuergröße angeordnet ist.

20. Verwendung nach Anspruch 19, dadurch gekennzeichnet, daß die Meßsonde ein Glukose-, pH-, $O_2$- oder $CO_2$-Meßsonde ist.

21. Verwendung nach einem der Ansprüche 17 bis 20, dadurch gekennzeichnet, daß die einzelnen Tanks und Vorratsbehälter Einrichtungen zur Erfassung der Füll- und Verbrauchsmengen besitzen.

## Claims

1. Process for the production of cell mass and/or fermentation products under sterile conditions, in which the fermentation mixture, at least at times, is circulated and the metabolic products of the cultivated cells, and possibly cell mass, are separated, said process comprising the following steps:

   - charging the fermentation equipment with a sufficient amount of nutrient medium to start the desired cell culture;
   - sterilizing the apparatus and adjusting the desired concentration of the nutrient medium, which is lower than the specific nutrient medium concentration of the cell culture;
   - inoculating the nutrient medium with the starter culture and allowing undisturbed growth of the culture for a defined time, in a batchwise mode and with partial filling of the fermentation equipment;
   - increasing the concentration of the nutrient medium to the specific nutrient medium concentration of the cell culture, with simultaneous increase of the volume of the nutrient medium to the working volume of the fermentation apparatus and of the cell concentration;
   - effecting transition to continuous procedure with exchanging of nutrient medium, separation of the metabolic products as well as complete or partial cell recycling;
   - terminating the continuous procedure at a desired time and harvesting the cell mass under sterile conditions; and optionally
   - repeating one or all of the aforementioned steps in the order set forth.

2. Process according to claim 1,
characterized in that in the phase of medium exchange the amount of medium dispensed is increased with the exponential growth of the culture and is held constant when maximal cell separation is reached.

3. Process according to claim 1 or claim 2,
characterized in that the nutrient medium is dispensed under the control of a control parameter whose value is continuously determined.

4. Process according to claim 3,
characterized in that as control parameter the pH value, the $CO_2$ concentration or the $O_2$ concentration is used.

5. Process according to claim 4,
characterized in that the pH value is determined in the fermentation apparatus and is held constant by addition of a base of defined concentration.

6. Process according to claim 5,
characterized in that an amountof nutrient medium is added which is equivalent to the amount of base consumed, said amount being varied by a dispensing factor k proportional to the growth rate of the cell culture in the growth phase.

7. Process according to claim 3,
characterized in that spent nutrient medium is replaced according to an envelope curve specific for the cell culture which takes into account the cell mass present, the growth rate and the culture conditions.

8. Process according to any one of claims 1 to 7,
characterized in that it is carried out continuously.

9. Process according to claim 8,
characterized in that the cell culture is adjusted to a required growth rate by variation of the harvest proportion or the cell recycling.

10. Process according to any one of claims 1 to 9,
characterized in that the metabolic products and the cell mass are separated by centrifugation.

11. Process according to any one of claims 1 to 10,
characterized in that the fermentation apparatus is initially charged with a nutrient medium concentrate.

12. Process according to claim 11,
characterized in that the setting of the initial nutrient medium concentration is by cold injection of sterile water.

13. Process according to any one of claims 1 to 12,
characterized in that the nutrient medium is added as concentrate.

14. Process according to any one of claims 1 to 13,
characterized in that the cell mass is partially recycled in the harvest phase.

15. Process according to any one of claims 1 to 14,
characterized in that in the cultivation of aerobic cells air enriched with pure oxygen is supplied to the cell culture.

16. Use of the process according to any one of claims 1 to 15 for the recovery of lactic acid bacteria and/or lactic acid.

17. Use of an apparatus for the production of cell mass and/or fermentation products comprising a sterilizable fermentation kettle, at least one tank for receiving and thermal sterilization of a medium component not sterilizable by filtration and/or at least one supply container for receiving a medium sterilizable by filtration and having a sterile filter for continuous production of a sterile medium component and a sterilizable circulation connected to the fermentation kettle and having means for separating spent nutrient medium and metabolic products, where the sterilizable circuit includes a centrifuge and a retaining section is provided between the fermenter and centrifuge the retaining section having a length sufficient to consume nutrient substances still contained in the nutrient medium, for carrying out the process according to claim 1.

18. Use according to claim 17, characterized in that the centrifuge is a steam-sterilizable self-desludging centrifuge with adjustable desludging amounts.

19. Use according to claim 17 or claim 18, characterized in that in the fermentation kettle a measuring probe is arranged for detecting a process control parameter.

**20.** Use according to claim 19, characterized in that the measuring probe is a glucose, pH, $O_2$ or $CO_2$ measuring probe.

**21.** Use according to any of claims 17 to 20, characterized in that the individual tanks and supply containers have means for detecting the level and consumption amounts.

**Revendications**

**1.** Procédé de production de masse cellulaire et/ou de produits de fermentation dans des conditions stériles, où le mélange de fermentation est envoyé au moins par intermittence en circuit et les produits du métabolisme des cellules cultivées et le cas échéant la masse cellulaire sont séparés, lequel procédé présente les étapes suivantes :

- charger dans l'installation de fermentation une quantité de milieu nutritif suffisante pour le démarrage de la culture cellulaire désirée ;
- stériliser l'installation ainsi qu'ajuster la concentration désirée en milieu nutritif, laquelle est inférieure à la concentration spécifique en milieu nutritif de la culture cellulaire ;
- ensemencer le milieu nutritif au moyen de la culture d'ensemencement et laisser croître la culture sans perturbations pendant une durée définie selon un régime en discontinu et en remplissant partiellement l'installation de fermentation ;
- amener la concentration en milieu nutritif à la concentration en milieu nutritif spécifique de la culture cellulaire tout en amenant le volume de milieu nutritif au volume de fonctionnement de l'installation ainsi que de la concentration cellulaire ;
- passer à un régime en continu avec remplacement du milieu nutritif et séparation des produits du métabolisme ainsi que recyclage cellulaire total ou partiel ;
- interrompre le régime en continu à un délai souhaité et récolter la masse cellulaire dans des conditions stériles et le cas échéant
- répéter tout ou partie des étapes préalablement citées dans l'ordre cité.

**2.** Procédé selon la revendication 1, caractérisé en ce qu'au cours de la phase de remplacement du milieu la quantité de milieu ajoutée est augmentée parallèlement à la croissance exponentielle de la culture et est maintenue constante lors de l'atteinte de la séparation cellulaire maximale.

**3.** Procédé selon la revendication 1 ou 2, caractérisé en ce que l'adjonction du milieu nutritif est commandée par le biais d'un paramètre-guide dont la valeur est déterminée continûment.

**4.** Procédé selon la revendication 3, caractérisé en ce qu'on utilise comme paramètre-guide le pH, la concentration en $CO_2$ ou la concentration en $O_2$.

**5.** Procédé selon la revendication 4, caractérisé en ce que le pH est déterminé dans l'installation de fermentation et maintenu constant par l'adjonction d'une base de concentration définie.

**6.** Procédé selon la revendication 5, caractérisé en ce qu'on ajoute une quantité de milieu nutritif équivalente à la quantité de base consommée et qui varie au cours de la phase de croissance de la culture cellulaire d'un facteur de dosage k proportionnel à la vitesse de croissance.

**7.** Procédé selon la revendication 3, caractérisé en ce qu'on remplace le milieu nutritif consommé selon une courbe-enveloppe spécifique de la culture cellulaire et qui tient compte des masses cellulaires présentes, de la vitesse de croissance et des conditions de culture.

**8.** Procédé selon une des revendications 1 à 7, caractérisé en ce qu'il est mis en oeuvre en continu.

**9.** Procédé selon la revendication 8, caractérisé en ce que la culture cellulaire est ajustée à une vitesse de croissance désirée par variation de la proportion récoltée ou du recyclage cellulaire.

**10.** Procédé selon une des revendications 1 à 9, caractérisé en ce que les produits du métabolisme et la masse cellulaire sont séparés par centrifugation.

**11.** Procédé selon une des revendications 1 à 10, caractérisé en ce que l'installation de fermentation est chargée initialement de milieu nutritif concentré.

12. Procédé selon la revendication 11, caractérisé en ce que l'ajustement de la concentration initiale en milieu nutritif s'effectue par pulvérisation à froid d'eau stérile.

13. Procédé selon une des revendications 1 à 12, caractérisé en ce que le milieu nutritif est ajouté sous forme de concentré.

14. Procédé selon une des revendications 1 à 13, caractérisé en ce qu'une partie de la masse cellulaire est recyclée au cours de la phase de récolte.

15. Procédé selon une des revendications 1 à 14, caractérisé en ce que de l'air enrichi en oxygène pur est envoyé à la culture cellulaire lors de la culture de cellules aérobies.

16. Application du procédé selon une des revendications 1 à 15 à l'obtention de bactéries lactiques et/ou d'acide lactique.

17. Utilisation d'un dispositif de production de masse cellulaire et/ou de produits de fermentation comportant un fermenteur stérilisable, au moins un réservoir de recueil et de stérilisation thermique d'un composant du milieu non susceptible de filtration stérile et/ou au moins un récipient de stockage d'un composant du milieu susceptible de filtration stérile avec filtre stérile en vue de la production continue d'un composant de milieu stérile, ainsi qu'un circuit stérilisable connecté au fermenteur et doté d'appareils de séparation du milieu nutritif consommé et des produits du métabolisme, le circuit stérilisable présentant une centrifugeuse et un tronçon d'arrêt dont la longueur suffit à la consommation des nutriments encore contenus dans le milieu nutritif étant prévu entre le fermenteur et la centrifugeuse, pour la mise en oeuvre du procédé selon la revendication 1.

18. Utilisation selon la revendication 17, caractérisée en ce que la centrifugeuse est une centrifugeuse stérilisable à la vapeur, auto-décolmatante, à quantités de décolmatage réglables.

19. Utilisation selon la revendication 17 ou 18, caractérisée en ce qu'une sonde de mesure destinée à saisir une grandeur de commande du processus est disposée dans le fermenteur.

20. Utilisation selon la revendication 19, caractérisée en ce que la sonde de mesure est une sonde de mesure du glucose, du pH, de $O_2$ ou de $CO_2$.

21. Utilisation selon une des revendications 17 à 20, caractérisée en ce que les différents réservoirs et récipients de stockage possèdent des appareils destinés à saisir les quantités introduites et consommées.

# FIG.1

Zellmassenausbeute pro Zelt für
Staphylococcus carnosus Sc1

Keimz./Ansatz 4000l (log) — Zellmassenausbeute (%)

—+— Keimz./Ansatz 4000l ■ Zellmassenausbeute

EP 0 487 867 B1

# FIG.2

| Fermentervolumen (1)<br>Mediumart. | 4.300<br>Zuchtmedium | 1.000<br>Mediumkonzentrat |
|---|---|---|
| Aufheizzeit bis 121 'C (min) | 80 | 45 |
| Sterilisationszeit (min) | 30 | 30 |
| Kühlzeit bis 30 'C (min) | 95 | 80 |
| Gesamtzeit (min) | 205 | 155 |
| Anteile Zeit (%) | 100 | 75,6 |
| Bedarf Stadtgas (m³) | 146,9 | 40 |
| Anteile Stadtgas (%) | 100 | 27,2 |
| Bedarf Kühlwasser (m³) | 28 | 7 |
| Anteile Kühlwasser (%) | 100 | 25 |
| Bedarf Strom (Verbrauchseinh. | 37 | 10 |
| Anteile Strom (%) | 100 | 27 |

# FIG.3

Abhängigkeit der mittleren Wachstumsrate u von der Ausgangsglucosekonz. im Medium für Lactobacilus curvatus S.3

# FIG.4

Verlauf der spez Wachstumsrate μ
bei linearem und exp.Zellwachstum

(•E09) Keimzahl (KbE/ml)      spez.Wachstumsrate μ

5,5 Stunden zusätzlicher Medienverbrauch
für Erhaltungsstoffwechsel

Stunden

—•— Keimzahl 1 (KbE/ml)     —+— spez.Wachstumsr.μ 1

—*— Keimzahl 2 (KbE/ml)     —▫— spez.Wachstumsr.μ 2

EP 0 487 867 B1

FIG. 5

Abb.1 FERMENTER MIT ZELLRÜCKFÜHRUNG
ÜBER SEPARATOR

▲ MEDIENFLUSSRICHTUNG

24

EP 0 487 867 B1

ABB. 2 STEUERUNG FERMENTER MIT ZELLRÜCKFÜHRUNG ÜBER SEPARATOR

**FIG. 6A** SIGNALFLUSSRICHTUNG

Diagram: STEUERSCHRANK FERMENTER F1 SF1 — SETPOINT-STEUERUNG FERMENTER F1 F1 SPS, with columns SIGNALEINGANG IN STEUERSCHRANK, SPS-VENTILSTEUERUNG VS, MESS-U.REGEL-EBENE SPS, SPS-COMPUTERINTERFACE C1. Signal labels: V1, V2, V3, V4, V5, V7, V8, V9, V10, V12, V13, V15, V16, V18, V19, P1, P2, P3, RF 1, MF 1, MF 2, IDM 1, IDM 2, S/LIRA, L/LIRA, A/LIRA, B1/LIRA, F1/TAICR, F1 pH/QAICR, F1FH/QAICR, F1O2/QAICR, F1CO2/QAICR, F1/ WICA, F1/SAICR, F1/ZULUFTR. Bottom labels: VON-ZU FERMENTER F1, MESSVER-STÄRKER MV, REGLER R, ANALOG ISTWERTE, ANALOG SOLLW. RS 232.

25

FIG. 6B

PROZESSLEITSYSTEM PLS
RECHNEREINHEIT RE

PLS -SIGNAL EIN -AUSGANG

· V1
· V2
· V3

· V5

· V7

· V9
· V10

RS 232.

· P1
· P2
· P3

· IDM1
· IDM2
· S/LIRA
· L/LIRA
· A/LIRA
· B1/LIRA
· F1/TAICR
· F1pH/QAICR
· F1EH/QAICR
· F1O$_2$/QAICR
· F1CO$_2$/QAICR
· F1/WICA
· F1/SAICR
· F1/ZULUFTR.

VENTIL-UND PUMPEN-
STEUERUNG DIGITAL EIN/AUS

ANALOG ISTWERTE
ERFASSUNG

ANALOG SOLLWERTE
VORGABE

BILDSCHIRM Bi

TASTATUR TA

DRUCKER Du

SOFTWARE
PROGRAMM PROZESSMANAGEMENT

RECHNER
HARDWARE

# FIG.7

Abb.     VP 29.05.90 PEB Bild 1/Korr.
Korr.zw.Ferm.vol.,Trm.im Ans.,Glu.konz.,
Glum.,Protm.,Kz.im Ans.,Ges.lact.

Legend:
- Ferm.vol.(*100)(l)
- Trm. im Ansatz (kg)
- Kelmz.im Ans.(KbE)
- Glu.konz. (g/l)
- Glu.menge (*10)(kg)
- Prot.menge (*10)(kg)
- Gesamtlactat (g/l)

Vol./Trm./Gluc.k./Glucm./Protm./Lact.k.        Kelmzahl

Stunden

1  Batch-Phase    2  Fed-batch-Phase    3  ZRF mit Fed-batch-Phase    4  ZRF mit Split-b.-fed-batch Phas
Schritt 2         Schritt 3            Schritt 4                     Schritt 5

EP 0 487 867 B1

# FIG. 8

EP 0 487 867 B1

Abb. y32   VP 29.05.90 PEB Bild 2/Korr.
Korr.zw.Glucosemenge,Glucosekonz.
Gesamtlactatkonz.und Verbrauch an NaOH

Glucm./Gluckonz./Lactkonz./NaOH-Verbr.

Schnitt 2

Schnitt 3   Schnitt 4   Schnitt 5

—▢— Glu.konz. (g/l)

—✕— Glu.menge (·10)(kg)

—△— Gesamtlactat (g/l)

—✳— NaOH-Verbr.(·10)(kg)

Abb.   VP 24.07.90 LAB Bild 1/Korr.
Korr.zw.Ferm.vol.,Trm.im Ans.,Glu.konz.,
Glum.,Protm.,Kz.im Ans.,Ges.,lact.

FIG.9

## FIG.10

Abb.    VP 24.07.90 LAB Bild 2/Korr.
Korr. zw. Glucosemenge,Glucosekonz.
Gesamtlactatkonz.und Verbrauch an NaOH

Glucm./Gluckonz./Lactkonz./NaOH-Verbr.

Schritt 2

Schaltschwelle
NaOH-Verbrauch

Schritt 3

Schritt 4

Schritt 5

Batchzeit (h)

—◻— Glu.konz. (g/l)          —✕— Glu.menge (*10)(kg)

—◆— NaOH-Ver.(*10)(kg)      —△— Gesamtlactat (g/l)

2 Batch-Phase; 3 Fed-batch-Phase; 4 ZRF; 5 Ernte;

EP 0 487 867 B1